# EUROPEAN PATENT APPLICATION

(11) **EP 4 177 344 A1**
(43) Date of publication of application: **10.05.2023**
(21) Application number: 21837627.5
(22) Date of filing: 06.07.2021
(51) Int. Cl.: C12N 15/113, C12N 15/10, C12N 9/22, C07K 14/74, C12N 5/0783, A61K 35/17, A61K 48/00

(54) **NOVEL TRANSPLANTATION CELLS HAVING REDUCED IMMUNOGENICITY**

(30) Priority: 06.07.2020 KR 20200082748
(71) Applicant: GC Cell Corporation, Gyeonggi-do 16924 (KR)
(72) Inventor: KIM, Hyun Ah, Yongin-si Gyeonggi-do 16924 (KR); KIM, Seung Min, Yongin-si Gyeonggi-do 16924 (KR); HER, Jung Hyun, Yongin-si Gyeonggi-do 16924 (KR); CHO, Sunglim, Yongin-si Gyeonggi-do 16924 (KR); KIM, Hyojin, Yongin-si Gyeonggi-do 16924 (KR); LIM, Hoyong, Yongin-si Gyeonggi-do 16924 (KR); CHO, Sungyoo, Yongin-si Gyeonggi-do 16924 (KR); HWANG, Yu Kyeong, Yongin-si Gyeonggi-do 16924 (KR)
(74) Representative: Schnappauf, Georg
(86) International application number: PCT/KR2021/008554
(87) International publication number: WO 2022/010220

(57) **Abstract**

The present invention relates to a composition for inhibiting immunogenicity of mammalian cells, and a method of producing hypoimmunogenic mammalian cells using the same. The cells of the present invention which are allogeneic transplanted for disease treatment, such as stem cells or immune cells, may be used as an effective cell therapy product having a long-term activity with minimized immune rejection in the recipient's body.

## Description

### Technical Field

The present invention relates to a novel composition for cell therapy with inhibited immunogenicity for efficient allogeneic transplantation, which is obtained by gene editing.

### Background Art

Natural killer (NK) cells are lymphocytes that account for about 10% of blood cells and play an important role in immune responses. NK cells perform several functions, and in particular, they have the ability to kill cancer cells or cells infected with exogenous pathogens, thereby eliminating abnormal cells that can be lesioned.

Most NK cells *in vivo* normally exist in an inactivated state, but activated NK cells are required to use them for therapeutic purposes, and thus studies on activating NK cells from normal blood or patient blood have been actively conducted. It has been found that, when NK cells are activated *in vitro,* the NK cells exhibit high cytotoxicity and thus may be used for immune cell therapy. In addition, it has been found that, when NK cells activated *in vitro* are administered to patients with various cancers, particularly blood cancer such as leukemia, by allogeneic bone marrow transplantation, they exhibit a therapeutic effect *(*Blood Cells Molecules & Disease, 33: p261-266, 2004).

Meanwhile, current anticancer immunotherapy using NK cells is focused on NK cell inhibitory receptors. That is, it is intended to increase the activity of NK cells by blocking the function of inhibitory receptors that inhibit NK cell activity. For this purpose, a method of using allogeneic NK cells from a donor that does not match the major histocompatibility complex (MHC) of the recipient or blocking the function of inhibitory receptors by antibodies is mainly used. Since the donor's and recipient's MHC Class I genotypes are different from each other and the inhibitory receptors (KIR, etc.) of the donor NK cells do not recognize the recipient's MHC Class I, the activity of allogeneic NK cells is not inhibited. Accordingly, it is expected that allogeneic NK cells may be free from the inhibition of MHC Class I activity, which may still exist in cancer cells, and thus they may exhibit more effective anticancer activity than patients' own NK cells that are not free from the inhibition of MHC Class I activity. In fact, the fact that allogeneic NK cells have many advantages for anticancer therapy was first known from the fact that allogeneic hematopoietic stem cell transplantation (HSCT) was effective in treating blood cancer patients. Additional studies revealed that a significant portion of the graft-versus-leukemia (GVL) response in this allogeneic hematopoietic stem cell transplantation is due to NK cells. Based on this fact, many attempts have been made to administer NK cells, obtained by culturing and activating *in vitro* a large amount of allogeneic NK cells isolated and purified from healthy donors, to cancer patients, and these NK cells exhibited effective anticancer activity against acute myeloid leukemia (AML), multiple myeloma (MM), and the like. However, it was reported that allogeneic hematopoietic stem cell transplantation with completely mismatched MHC Class I does not exhibit expected anticancer activity in some patients in many cases, and had serious side effects such as infection by immunosuppressants included in the "conditioning regimen". Moreover, in the case of NK cells, recent studies have revealed that the interaction ("education" or "licensing' process") between the inhibitory receptor and the target cell MHC Class I is required to obtain sufficient anticancer activity during differentiation of NK cells. Thus, it is expected that the MHC Class I of the donor and the MHC Class I of the recipient should match to some extent in order to obtain mature NK cells having sufficient anticancer activity. Therefore, due to this influence, many attempts are currently being made to treat hematological cancer using haploidentical hematopoietic stem cell transplantation with a match in one of different MHC Class I genotypes and NK cells, and it has been found that clinical prognosis resulting therefrom is relatively excellent. In addition, when allogeneic immune cells are used as a cell therapy product, side effects such as graft-versus-host disease (GVHD) that attack the recipient's own cells inevitably occur, whereas, in the case of allogeneic NK cells, this problem was rarely reported. It is understood that, unlike cancer cells, normal cells hardly express ligands for NK cell activation receptors and thus do not induce NK cell activation. Currently, studies have been actively conducted to use allogeneic NK cells as a cell therapy product for other solid cancers in addition to blood cancer (Kim, HS, Hanyang Med Rev, 33:59-64, 2013).

Another problem with allogeneic NK cell transplantation is that the transplanted NK cells do not persist for a long time in the recipient. To overcome this problem, a method of periodically adding the cytokine IL-2 capable of inducing NK cell proliferation after transplantation was attempted. However, IL-2 can become a big problem because it can expand regulatory T cells (Tregs) that suppress anticancer immune responses, in addition to NK cells (Romagne F, Vivier E.,F1000 Med Rep, 3:9, 2011; Waldmann TA., Nat Rev Immunol, 6:595-601, 2006).

Through the specification, a number of publications and patent documents are referred to and cited. The disclosure of the cited publications and patent documents is incorporated herein by reference in its entirety to more clearly describe the state of the related art and the present invention.

### DISCLOSURE

### Technical Problem

The present inventors have made extensive studies to develop an effective cell therapy product exhibiting a long-term activity while showing minimized immune rejection in a patient's body. As a result, the present inventors have found that, when gene editing components targeting a type II HLA gene or an activating protein thereof, specifically a specific portion of CIITA, which is an immunogenicity inducer, and a specific portion of B2M gene, are introduced into therapeutic cells, the efficiency for inhibiting the immunogenicity of the cells may be maximized, so that the cells could be used as an excellent cell therapy product for allogeneic transplantation, thereby completing the present invention.

Therefore, an object of the present invention is to provide a composition for inhibiting the immunogenicity of mammalian cells and hypoimmunogenic mammalian cells produced using the same.

Another object of the present invention is to provide a method for producing hypoimmunogenic mammalian cells.

Other objects and advantages of the present invention will become more apparent from the following detailed description of the invention, the appended claims and the accompanying drawings.

### Technical Solution

According to one aspect of the present invention, the present invention provides a composition for inhibiting immunogenicity of mammalian cells comprising, as an active ingredient, a nucleic acid molecule that inhibits expression of type II HLA protein.

The present inventors have made extensive studies to develop an effective cell therapy product exhibiting a long-term activity while showing minimized immune rejection in a patient's body. As a result, the present inventors have found that, when gene editing components targeting a type II HLA gene or an activating protein thereof, specifically a specific portion of CIITA, which is an immunogenicity inducer, and a specific portion of B2M gene, are introduced into therapeutic cells, the efficiency for inhibiting the immunogenicity of the cells may be maximized, so that the cells could be used as an excellent cell therapy product for allogeneic transplantation.

As used herein, the term "nucleic acid molecule" has a meaning comprehensively including DNA (gDNA and cDNA) and RNA molecules, and nucleotides, which are the basic structural units in nucleic acid molecules, include not only natural nucleotides, but also analogues in which sugar or base sites are modified (Scheit, Nucleotide Analogs, John Wiley, New York(1980); Uhlman and Peyman, Chemical Reviews, 90:543-584(1990)).

As used herein, the term "inhibition of expression" means reducing the activity or expression of a target gene so that the activity or expression of the target gene becomes undetectable, or the target gene may be present at an insignificant level, or the biological function of the target may be significantly reduced. The nucleic acid molecule for inhibiting expression according to the present invention is specifically a nucleic acid molecule complementary to the sequence of the gene of interest, and examples thereof include, but are not limited to, shRNA, siRNA, miRNA, guide RNA (gRNA) and an antisense oligonucleotide. In addition, any nucleic acid molecule known in the art may be used as a means for inhibiting gene expression.

According to a specific embodiment of the present invention, the nucleic acid molecule is a guide RNA (gRNA), which specifically recognizes a nucleotide sequence encoding the type II HLA protein or an activating protein thereof, or a nucleotide sequence encoding the gRNA.

As used herein, the term "guide RNA (gRNA)" refers to an RNA molecule used in a gene editing system that recognizes a target gene and induces a nuclease to specifically cleave the recognized site. A typical example of this gene editing system is a CRISPR (Clustered Regularly Interspaced Short Palindromic Repeats) system.

As used herein, "specifically recognizes" means that the gRNA may be selectively hybridized with a target nucleotide sequence by having a sequence complementary to the target nucleotide sequence. The term "complementary" means that the gRNA is complementary enough to selectively hybridize with a target sequence under certain annealing or hybridization conditions. The term "complementary" is meant to include both substantially complementary and perfectly complementary, and preferably means perfectly complementary. As used herein, the term "substantially complementary sequence" is meant to include a perfectly matching sequence but also a sequence partially mismatching with a comparison sequence, within a range where the gRNA may be annealed to the specific sequence and serve as gRNA.

When taking into consideration variations having biologically equivalent activity, the nucleotide sequence that is to be specifically recognized to inhibit expression thereof in the present invention is interpreted to include a sequence having substantial identity to the known sequence of the type II HLA gene. The term "substantial identity" means that a sequence has a homology of at least 70%, specifically at least 80%, more specifically at least 90%, most specifically at least 95%, when aligning the nucleotide sequence with any other sequence so as to correspond to each other as closely as possible and analyzing the aligned sequence using algorithms commonly used in the art. Alignment methods for sequence comparison are well-known in the art. Various methods and algorithms for alignment are described in Huang et al., Comp. Appl. BioSci. 8:155-65(1992) and Pearson et al., Meth. Mol. Biol. 24:307-31(1994). The NCBI Basic Local Alignment Search Tool (BLAST) (Altschul et al., J. Mol. Biol. 215:403-10(1990) is accessible through NCBI or the like, and may be used in conjunction with sequence analysis programs such as blastp, blasm, blastx, tblastn and tblastx on the Internet.

More specifically, the activating protein of the type II HLA protein is a transactivator for the type II HLA protein, even more specifically, a class II major histocompatibility complex transactivator (CIITA) protein.

According to a specific embodiment of the present invention, the composition further contains RNA-guided endonuclease or a nucleotide sequence encoding the RNA-guided endonuclease. "RNA-guided endonuclease" is an enzyme that is guided by gRNA recognizing a target gene site and cleaves a target gene, and this RNA-guided endonuclease may be delivered in the form of mRNA or protein, or may be delivered to a target cell by transformation with a vector loaded with DNA encoding the same. When an endonuclease in the form of protein is used, the endonuclease may form a ribonucleoprotein complex (RNP) complex with the guide RNA.

As used herein, the term "RNP complex" includes the guide RNA and the RNA-guided endonuclease as active ingredients, and the complex may recognize and bind to a target sequence and selectively nick or cleave the target sequence. The RNA complex may be, for example, a Cas9-gRNA complex, without being limited thereto

In one embodiment of the present invention, the RNA-guided endonuclease may be any one selected from the group consisting of Cas1, Cas1B, Cas2, Cas3, Cas4, Cas5, Cas6, Cas7, Cas8, Cas9, Cas10, Cas12a, Cas12b, Cas12c, Cas12d, Cas12e, Cas 13a, Cas 13b, Cas 13c, Cas 13d, Cpf1, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4 and MAD7. Specifically, the RNA-guided endonuclease is Cas9 (CRISPR associated protein 9), Cpf1 (CRISPR from Prevotella and Francisella 1), or MAD7.

As used herein, the term "nucleotide sequence" has a meaning comprehensively including DNA (gDNA and cDNA) and RNA molecules, Nucleotides, which are the basic structural units in nucleic acid molecules, include not only natural nucleotides, but also analogues in which sugar or base sites are modified. It will be apparent to those skilled in the art that the nucleotide sequence encoding the gRNA or RNA-guided endonuclease in the present invention is not limited to the nucleotide sequence set forth in the accompanying sequence listing. Mutation in a nucleotide sequence may not result in a change in a protein (e.g., an endonuclease), and examples of this nucleic acid sequence include all nucleic acid molecules containing functionally equivalent codons, codons encoding the same amino acids (by the degeneracy of codons), or codons encoding biologically equivalent amino acids.

According to a specific embodiment of the present invention, gRNA specifically recognizes a nucleotide sequence selected from the group consisting of SEQ ID NO: 20, SEQ ID NO: 22, SEQ ID NO: 24, SEQ ID NO: 27, SEQ ID NO: 29, SEQ ID NO: 30, SEQ ID NO: 40 and SEQ ID NO: 44, or a sequence complementary thereto.

According to a specific embodiment of the present invention, the composition of the present invention further contains a nucleic acid molecule that inhibits the expression of β2-microglobulin protein.

More specifically, the nucleic acid molecule is a guide RNA (gRNA), which specifically recognizes a nucleotide sequence encoding the β2-microglobulin protein, or a nucleotide encoding the gRNA.

More specifically, the guide RNA (gRNA) specifically recognizes a nucleotide sequence selected from the group consisting of SEQ ID NO: 1, SEQ ID NO: 8, SEQ ID NO: 9 and SEQ ID NO: 14, or a sequence complementary thereto.

According to a specific embodiment of the present invention, the composition further contains a nucleic acid molecule encoding a type I HLA protein. More specifically, the type I HLA protein is an HLA-E protein.

Another problem with allogeneic cell transplantation is that the transplanted therapeutic cells do not persist for a long period in the recipient. To overcome this problem, a method of periodically injecting specific cytokines capable of inducing cell proliferation after transplantation has been proposed, but cytokines such as IL-2 may cause adverse effects in anticancer therapy because they can amplify regulatory T cells (Tregs) that suppress anticancer immune responses (Romagne F, Vivier E., F1000 Med Rep, 3:9, 2011; Waldmann TA., Nat Rev Immunol, 6:595-601, 2006). Therefore, the present inventors attempted to introduce HLA-E, which can prevent cell death by stimulating the inhibitory receptor CD94/NKG2A on therapeutic cells such as immune cells or stem cells. As shown in Examples below, it was confirmed that introducing HLA-E may prevent the transplanted cells for treatment according to the present invention from being killed by the immune response of the recipient, indicating that the cells may exhibit a pharmacological effect while being maintained for a long time in the body of the recipient.

As used herein, the term "expression" means allowing a target cell to express an exogenous gene or artificially introducing an endogenous gene using a gene delivery system in order to increase the natural expression level of the endogenous gene so that the gene is replicable as an extrachromosomal factor or by chromosomal integration in subject's cells. Thus, the term "expression" has the same meaning as "transformation", "transfection" or "transduction".

As used herein, the term "gene delivery system" refers to any means for delivering a gene into a cell, and gene delivery has the same meaning as intracellular transduction of a gene. At the tissue level, the term "gene transfer" has the same meaning as the spread of a gene. Therefore, the gene delivery system of the present invention may be described as a gene transduction system and a gene spreading system.

In order to produce the gene delivery system of the present invention, the nucleotide sequence of the present invention is preferably present in a suitable expression construct. In the expression construct, the nucleotide sequence of the present invention is preferably operatively linked to a promoter. As used herein, the term "operatively linked" refers to a functional linkage between a nucleic acid expression regulatory sequence (e.g., a promoter, a signal sequence, or an array of transcription regulation factor binding sites) and another nucleic acid sequence, and through the linkage, the regulatory sequence regulates the transcription and/or translation of the other nucleic acid sequence. The promoter linked to the nucleotide sequence of the present invention is one that can regulate the transcription of the HLA-E gene by action specifically in animal cells, more specifically mammalian cells, includes, for example, promoters derived from mammalian viruses and promoters derived from mammalian cell genomes. Examples of the promoter include, but are not limited to, mammalian cytomegalovirus (CMV) promoter, adenovirus late promoter, vaccinia virus 7.5K promoter, SV40 promoter, HSV tk promoter, RSV promoter, EF1 alpha promoter, metallothionein promoter, beta-actin promoter, human IL-2 gene promoter, human IFN gene promoter, human IL-4 gene promoter, human lymphotoxin gene promoter, and human GM-CSF gene promoter.

The nucleotide sequence of the HLA-E gene may be applied to any gene delivery system used for ordinary gene introduction. Preferably, the nucleotide sequence of the HLA-E gene may be applied to plasmids, adenoviruses (Lockett LJ, et al., Clin. Cancer Res. 3:2075-2080(1997)), adeno-associated viruses (AAV) (Lashford LS., et al., Gene Therapy Technologies, Applications and Regulations Ed. A. Meager, 1999), retroviruses (Gunzburg WH, et al., Retroviral vectors. Gene Therapy Technologies, Applications and Regulations Ed. A. Meager, 1999), lentiviruses (Wang G. et al., J. Clin. Invest. 104(11):R55-62(1999)), herpes simplex virus (Chamber R., et al., Proc. Natl. Act. Sci USA 92:1411-1415(1995)), vaccinia viruses (Puhlmann M. et al., Human Gene Therapy 10:649-657(1999)), liposomes (Methods in Molecular Biology, 199, S.C. Basu and M. Basu (Eds.), Human Press 2002) or niosomes. Specifically, the gene delivery system of the present invention may be prepared by applying the nucleotide molecule of the present invention to a lentivirus.

In the present invention, when the gene delivery system is constructed based on a viral vector, the contacting step is performed according to a viral infection method known in the art. The infection of host cells using viral vectors are described in the above-mentioned cited documents.

According to a specific embodiment of the present invention, the cells into which the composition of the present invention is introduced are allogeneic or autologous cells for transplantation, more specifically allogeneic cells for transplantation.

More specifically, the cells are stem cells or immune cells.

As used herein, the term "stem cells" refers to undifferentiated cells that are in a stage before differentiation into each type of cells constituting a tissue, and collectively refer to cells having the capability to differentiate into a specific type of cells under a specific differentiation stimulus (environment). Unlike differentiated cells whose cell division was halted, the stem cells can produce the same cells as themselves by cell division (self-renewal), and when a differentiation stimulus is applied, the stem cells have the plasticity of differentiation that they can be differentiated into various types of cells depending on the nature of the stimulus.

The stem cells to which the present invention is applied are not limited, and cells having characteristics of stem cells, that is, undifferentiation, indefinite proliferation, and ability to differentiate into a specific type of cells, are cells to which the present invention may be applied. Specifically, the stem cells that are used in the present invention are mesenchymal stem cells or pluripotent stem cells.

As used herein, the term "mesenchymal stem cells" refers to stem cells having multipotency to differentiate into adipocytes, osteocytes, chondrocytes, muscle cells, nerve cells, and cardiomyocytes. Mesenchymal stem cells are identified through a morphological characteristic such as a spindle shape and the expression levels of the basic cell surface markers CD73(+), CD105(+), CD34(-), and CD45(-). One of the important characteristics of mesenchymal stem cells is that they also have a function of regulating immune responses. It has been reported that mesenchymal stem cells have the ability to differentiate into bone tissue, central nervous system tissue, skin tissue and muscle tissue, and thus may be used for regeneration of these tissues, which have been physically lost, through appropriate differentiation inducers, and exhibit a strong immunomodulatory effect by inhibiting the proliferation, function and activity of T-cells, B-cells, natural killer (NK) cells and dendritic cells, indicating that they may also be applied for the treatment of various diseases such as transplant rejection, autoimmune diseases, inflammatory diseases and allergic diseases, which are caused by unwanted or excessive immune responses. Accordingly, when the present invention is applied to mesenchymal stem cells, it may be applied for regenerative treatment in various degenerative diseases or treatment of autoimmune/inflammatory diseases.

As used herein, the term "pluripotent stem cells" refers to stem cells that have more developed than a fertilized egg and are capable of differentiating into cells constituting endoderm, mesenchymal and ectoderm. According to a specific embodiment of the present invention, the pluripotent stem cells that are used in the present invention are embryonic stem cells (ESCs), embryonic germ cells, embryonic carcinoma cells, or induced pluripotent stem cells (iPSCs). More specifically, the pluripotent stem cells are induced pluripotent stem cells.

As used herein, the term "induced pluripotent stem cells" is a type of pluripotent stem cells artificially derived from non-pluripotent cells (e.g., somatic cells) by insertion of specific genes related to an undifferentiated or pluripotent phenotype. In the art, induced pluripotent stem cells are considered as having the same phenotype, physiological characteristics and developmental characteristics as natural pluripotent stem cells (e.g., embryonic stem cells) in that they have stem cell gene and protein expression, chromosomal methylation, doubling time, embryoid body formation, teratoma formation, viable chimera formation, hybridization ability and differentiation ability.

As used herein, the term "immune cells" refers to any cells involved in the initiation or promotion of immune response, and more specifically refers to immune effector cells. Immune cells include, but are not limited to, for example, T cells, B cells, natural killer (NK) cells, natural killer T (NKT) cells, and mast cells. More specifically, the immune cells are natural killer cells.

When the present invention is applied to immune cells, it may be applied for the treatment of tumors and infectious diseases. The NK cells produced by the method according to the present invention may be applied to all types of tumors, including solid cancer and blood cancer. Unlike blood cancer, solid cancer refers to cancer formed as a lump in an organ, and includes cancers occurring in most organs. Tumors that may be treated using the NK cells according to the present invention are not specially limited and include gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, lymphoma, etc., without being limited thereto. Infectious diseases that can be treated using the immune cells of the present invention are diseases caused by infection with viruses or pathogens, and include all diseases that may be caused by infection through respiratory organs, blood, skin contact, etc. These infectious diseases include, but are not limited to, for example, hepatitis B and C, human papilloma virus (HPV) infection, cytomegalovirus infection, viral respiratory diseases, and influenza.

As used herein, the term "treatment" refers to (a) inhibiting the development of a disease, disorder or symptom; (b) alleviating the disease, disorder or symptom; or (c) eliminating the disease, disorder or symptom. When the stem cells into which the immunogenicity inhibitory composition of the present invention has been introduced are administered to a subject, they serves to inhibit the development of symptoms caused by excessive or unwanted immune responses, or eliminate, or alleviate the symptoms, and when the immune cells into which the immunogenicity inhibitory composition of the present invention has been introduced are administered to a subject, they serve to induce the death of cancer cells or infected cells, thereby inhibiting the development of symptoms caused by tumors or infectious diseases, or eliminating or alleviating the symptoms. Accordingly, the composition of the present invention may be a cell therapeutic composition for a disease by itself, or may be administered together with other pharmacological components and applied as a therapeutic adjuvant for the disease. Accordingly, as used herein, the term "treatment" or "therapeutic agent" includes the meaning of "therapeutic adjuvant" or "therapeutic adjuvant".

As used herein, the term "administration" or "administering" refers to directly administering a therapeutically effective amount of the composition of the present invention to a subject so that the same amount is formed in the subject's body.

As used herein, the term "therapeutically effective amount" refers to an amount of the composition that is sufficient to provide a therapeutic or prophylactic effect to a subject to which the composition of the present invention is to be administered, and thus the term is meant to include a "prophylactically effective amount".

As used herein, the term "subject" includes, without limitation, a human, mouse, rat, guinea pig, dog, cat, horse, cow, pig, monkey, chimpanzee, baboon or rhesus monkey. Specifically, the subject of the present invention is a human.

According to another aspect of the present invention, the present invention provides low-immunogenic mammalian cells produced using the above-described composition of the present invention.

Since the composition for inhibiting immunogenicity according to the present invention and the target cells into which the composition is to be introduced have already been described above, description thereof will be omitted to avoid excessive overlapping.

According to another aspect of the present invention, there is provided a method for producing low-immunogenic (hypoimmunogenic) mammalian cells comprising:
(a) inhibiting expression of a protein, selected from the group consisting of β2-microglobulin protein, type II HLA protein, an activating protein of type II HLA protein, and combinations thereof, in cells isolated from a mammalian subject; and
(b) introducing HLA-E gene into the cells.

According to the present invention, it has been found that, in the production of therapeutic cells for allogeneic transplantation according to the present invention, which have an activity which is maintained for a long time while having reduced immunogenicity, a process of inhibiting an immunogenic gene and then a process of introducing a gene for suppressing the attack of recipient's NK cells are sequentially performed, the resulting cells exhibit excellent growth characteristics, viability and activity.

As shown in the Examples to be described later, it was confirmed that, when the introduction of HLA-E was performed first, the yield of cells with a HLA-ABC-/HLA-E⁺ phenotype was very low, contrary to the expectation that even if the B2M gene is removed, the survival and growth of NK cells having the HLA-ABC-/HLA-E⁺ phenotype can be maintained during a culture process because the expression of HLA-E continues, and rather, the processes of removing the immunogenic gene and then introducing HLA-E showed a high yield in which cells having the HLA-ABC-/HLA-E⁺ phenotype are present at 96% or more until the end of culturing, indicating that this process sequence is the optimal process sequence for producing the low-immunogenic mammalian cells of the present invention.

According to a specific embodiment of the present invention, step (a) may be performed using a nucleic acid molecule for inhibiting expression of each of the above-described proteins and using the above-described endonuclease. Since these nucleic acid molecules and the endonuclease have already been described above, description thereof will be omitted to avoid excessive overlapping.

According to a specific embodiment of the present invention, step (b) is performed 1 to 5 days, more specifically 2 to 5 days, most preferably 3 days, after completion of step (a).

### Advantageous Effects

The features and advantages of the present invention are summarized as follows:
(a) The present invention provides a composition for inhibiting immunogenicity of mammalian cells and a method of producing hypoimmunogenic mammalian cells using the same.
(b) The cells of the present invention which are allogeneic transplanted for disease treatment, such as stem cells or immune cells, may be used as an effective cell therapy product having a long-term activity with minimized immune rejection in the recipient's body.

### Brief Description of Drawings

FIG. 1 shows the results of FACS performed to measure the expression level of HLA-ABC 3 days after gRNA treatment for B2M gene.
FIG. 2 shows the results of measuring the efficiency of knocking-out of B2M gene depending on the concentration of a shuttle used for delivery of a gene editing system of the present invention.
FIG. 3 shows changes in the HLA-ABC expression ratio depending on the culturing of the B2M gene knockout NK cells.
FIG. 4 shows results indicating that the viability of B2M- NK cells was increased by HLA-E transduction.
FIG. 5 shows the viability and growth rate of B2M knockout or HLA-E transformed NK cells.
FIG. 6 shows the Log-NK phenotype and production efficiency according to a production method.
FIG. 7 shows the expression levels of HLA-ABC and HLA-E depending on the culturing time of Log-NK.
FIG. 8 shows the expression levels of NK-specific markers at the end of culturing of Log-NK.
FIG. 9 shows cell viability and growth rate depending on the culturing of Log-NK.
FIG. 10 shows the results of evaluating the cancer cell line killing ability of Log-NK. FIG. 10a shows the degree of lysis of the K562 cancer cell line by wild-type NK cells or Log-NK cells, and FIG. 10b shows changes in the expression levels of cytokines in wild-type NK cells and Log-NK cells upon contact with K562 target cells.
FIG. 11a shows the results of observing the proportion of wild-type NK cells lysed by CD8(+) T cells using calcein AM, and FIG. 11b shows the results of observing the proportions of wild-type NK cells and Log-NK lysed by CD8(+) cells for a long period of time using CFSE.
FIG. 12 shows the results of flow cytometry performed to confirm the inhibition of expression of the type II HLA gene in NK cells by CIITA gene knockout.
FIG. 13 shows the results of flow cytometry performed to confirm the inhibition of expression of the type II HLA gene in NK cells at each time point of culture after CIITA gene knockout.

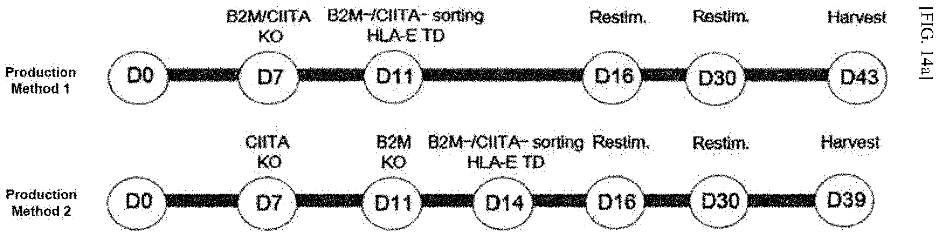

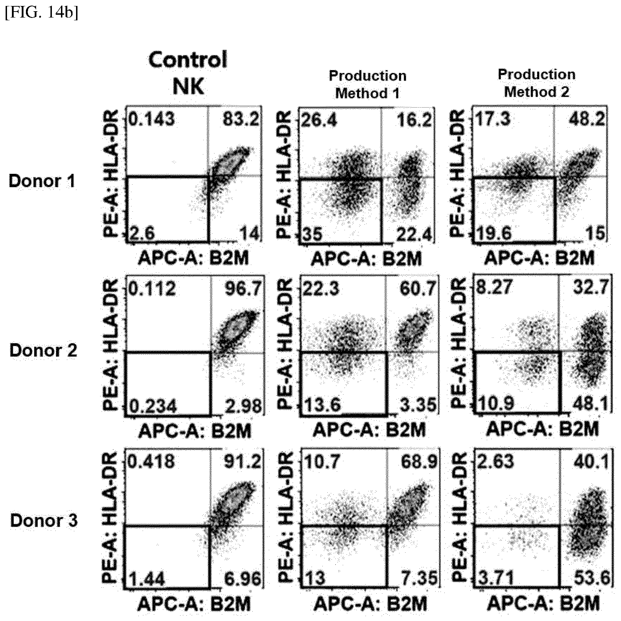

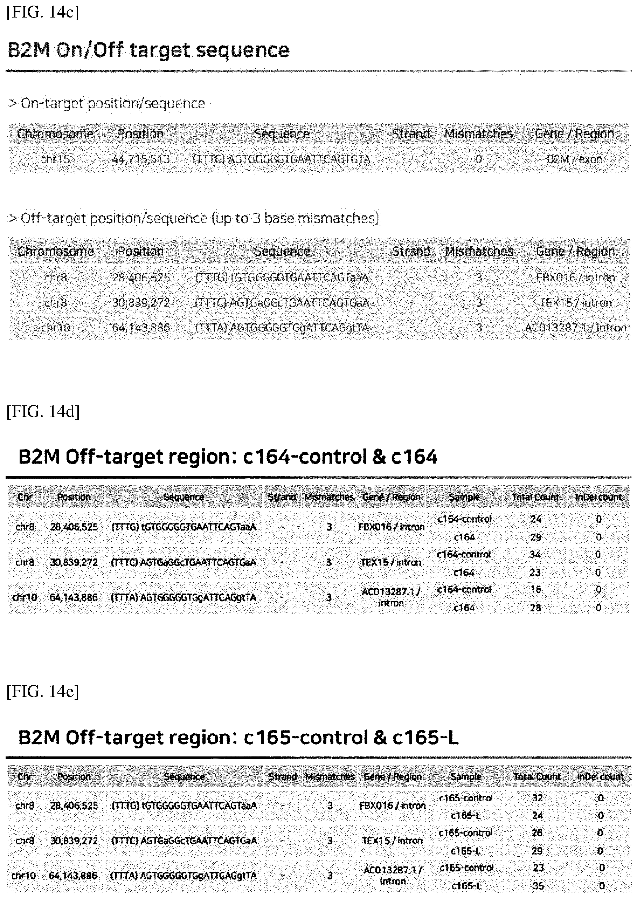

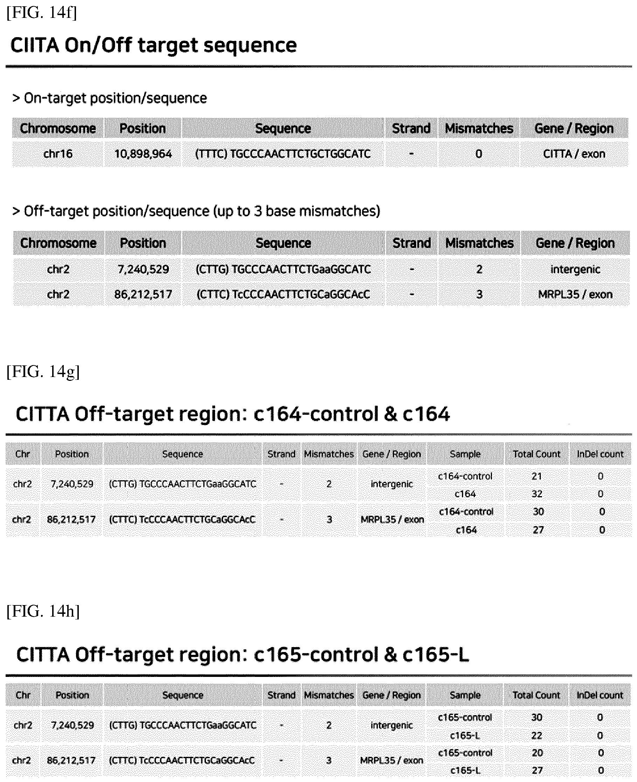

FIG. 14a is a schematic view summarizing a process for producing NK cells from which B2M and CIITA genes have been simultaneously removed, and FIG. 14b shows the efficiency of double knockout of B2M and CIITA in NK cells produced by each method shown in FIG. 14a. FIGS. 14c and 14f show the sequences of predicted on-target and off-target regions of gRNA in B2M and CIITA genes, respectively. FIGS. 14d and 14e show that no insertions/deletions occurred in three predicted off-target regions of B2M. FIGS. 14g and 14h show that no insertions/deletions occurred in two predicted off-target regions of CIITA.
FIG. 15 shows the expansion rate and viability of Log-NK-CIITA KO cells produced by each of Production Method 1 (FIG. 15a) and Production Method 2 (FIG. 15b) shown in FIG. 14a.
FIG. 16 shows the results of confirming the phenotype of Log-NK-CIITA KO cells during the culture period. FIG. 16a shows the inhibition of expression of HLA-ABC and HLA-DR/DP/DQ, and FIG. 16b shows the expression of HLA-E.
FIG. 17 shows the results of measuring the cancer cell killing ability of Log-NK-CIITA KO cells using each of Calcein-AM (FIG. 17a) and CFSE (FIG. 17b) cell staining methods.
FIG. 18 shows the results of confirming the efficiency of knockout of B2M (FIG. 18a) and CIITA (FIG. 18b) genes by various gRNA delivery methods.
FIG. 19 shows the results of examining whether Log-NK-CIITA KO cells of the present invention can evade the attack of recipient's CD8(+) T cells (FIG. 19a) and CD4(+) T cells (FIG. 19b) of the recipient.

### Mode for Invention

Hereinafter, the present invention will be described in more detail with reference to examples. These examples are only for illustrating the present invention in more detail, and it will be apparent to those of ordinary skill in the art that the scope of the present invention according to the subject matter of the present invention is not limited by these examples.

### Examples

### Example 1: Knockout of B2M gene using gene scissors

Low-immunogenic NK cells were produced using cord blood natural killer (CBNK) cells isolated from umbilical cord blood donated by donors.

β2m, which is common to HLA-A, HLA-B and HLA-C, which are human leukocyte antigens (HLAs) present on the cell surface, is made by expression from the B2M gene. For the production of NK cells that do not express HLA-ABC, the desired portion of the B2M gene in the genome of NK cells was cut using gene scissors technology in the following manner to inhibit the expression of β2m.

First, a reaction solution of RNP (ribonucleoprotein), a complex of gRNA and nuclease, was prepared in a 1.5 mL centrifuge tube as described below and allowed to react at room temperature for 5 minutes or more, and then used within 60 minutes.

20 µL of each of gRNAs (synthesized by IDT, 10 µM) having different sequences for cleaving the *B2M* gene was mixed with 3.32 µL of 40 µM Cpf1 nuclease (Feldan Therapeutics) in 26.84 µL of 1 × PBS or with 4 µL of MAD7 nuclease (Feldan Therapeutics) in 26 µL of 1 × PBS, and then allowed to react at room temperature for 5 minutes or more to obtain an RNP reaction solution. The prepared RNP reaction solution could be used for up to 1 to 4 × 10⁶ cells, and in this Example, cells were counted using an ADAM cell counter system (NanoEntek) and then 2 x 10⁶ NK cells were used. 2 x 10⁶ NK cells were placed in a separate 1.5 mL centrifuge tube and centrifuged at 2,000 rpm for 3 minutes, and then the supernatant was removed. 500 µL of 1 × PBS was added thereto and then centrifuged at 2,000 rpm for 3 minutes, and the supernatant was removed, leaving only the cells. 2 µL of Feldan shuttle (Feldan Therapeutics, 5 µM) was added to 48 µL of αlpha MEM medium (Sigma, M8042) in another 1.5 mL centrifuge tube, and then 50 µL of the RNP reaction solution was added thereto and mixed. Then, the mixture solution was added to the NK cells from which the supernatant has been removed, and allowed to react at room temperature for 1 minute and 30 seconds in a stationary state. Next, 2 mL of Cellgro medium (Cellgenix, 20802-0500) containing 1% (v/v) of human plasma and 1000 IU hIL-2 (Proleukin Inj., Novartis Korea) was added thereto to suspend the NK cells which were then transferred and cultured in a 6-well plate.

After stationary culture for 72 hours, 2 × 10⁵ NK cells were harvested and centrifuged at 1,200 rpm for 5 minutes, and the medium was removed. Next, the cells were suspended in FACS buffer containing 2 mL of 2% FBS and centrifuged at 2,000 rpm for 3 minutes, and the supernatant was removed, leaving only the NK cells. Next, 100 µL of FACS buffer was added to the cells, and then antibodies for analysis as shown in Table 1 below were added to the cells and reacted at 4°C for 30 minutes. 2 mL of FACS buffer was added to the cells, followed by centrifugation at 2,000 rpm for 3 minutes. The supernatant was removed, and the NK cells were fixed by adding 300 µL of a fixation (BD, 554655) solution. Then, expression on the stained NK cell surface was analyzed using the flow cytometer LSRFortessa (BD Bioscience).

As guide RNAs (gRNAs) capable of cleaving the *B2M* gene using Cpf1 or MAD7 nuclease, gRNA candidate sequences selected using the benchling (ttps://benchling.com) and CRISPR RGEN Tools (www.rgenome.net) program are listed in Table 2 below. The knockout efficiency of each gRNA was evaluated by analyzing HLA-ABC expression by FACS.

**[Table 1] Antibody for FACS analysis for NK cell phenotype**

| Marker | Antibody information (fluorescent/manufacturer/catalog No.) | Amount of antibody used |
|---|---|---|
| Human CD56 | APC / BD Bioscience / 555518 | 5 µL |
| Human HLA-ABC | BV421 / BD Bioscience / 565332 | 0.5 µL |
| Human CD3 | FITC / BD Bioscience / 555332 | 5 µL |
| 7-AAD | PerCp-Cv5.5 / Beckmen Coulter / A07704 | 5 µL |

**[Table 2] gRNA candidate sequences targeting B2M**

| SEQ ID NO. | Sequence | Length (bp) | Nuclease | Cleavage position | B2M knockout % (FACS) | Remarks |
|---|---|---|---|---|---|---|
| 1 | ATCCATCCGACATTGAAGT T | 20 | Cpf1 | Exon 2 | 23 | |
| 2 | ATCCATCCGACATTGAAGT TG | 21 | Cpf1 | Exon 2 | 9 | Sequence 1+1nt |
| 3 | ATCCATCCGACATTGAAGT TGAC | 23 | Cpf1 | Exon 2 | 0.8 | Sequence 1+2nt |
| 4 | TTAGAGTCTCGTGATGTTT AAG | 22 | Cpf1 | Promoter | 0.5 | |
| 5 | CCGAUAUUCCUCAGGUAC UCCA | 22 | Cpf1 | Exon 2 | 5 | |
| 6 | ATATAAGTGGAGGCGTCGC GC | 21 | Cpf1 | Exon 1 | 1.1 | |
| 7 | ATATAAGTGGAGGCGTCGC GCTG | 23 | Cpf1 | Exon 1 | 0.6 | Sequence 6+2nt |
| 8 | AGTGGGGGTGAATTCAGTG TA | 21 | Cpf1/ MAD7 | Exon 2 | 69.5 | |
| 9 | AGTGGGGGTGAATTCAGTG TAGT | 23 | Cpf1 | Exon 2 | 77 | Sequence 8+2nt |
| 10 | CTGAATTGCTATGTGTCTG GG | 21 | Cpf1 | Exon 2 | 3 | |
| 11 | CTGAATTGCTATGTGTCTG GGTT | 23 | Cpf1 | Exon 2 | 0.5 | Sequence 10+2nt |
| 12 | CTCACGTCATCCAGCAGAG AA | 21 | Cpf1 | Exon 2 | 5 | |
| 13 | CTCACGTCATCCAGCAGAG AATG | 23 | Cpf1 | Exon 2 | 0.8 | Sequence 12+2nt |
| 14 | AGCAAGGACTGGTCTTTCT AT | 21 | Cpf1 | Exon 2 | 32 | |
| 15 | AGCAAGGACTGGTCTTTCT ATCT | 23 | Cpf1 | Exon 2 | 0.2 | Sequence 14+2nt |
| 16 | CATTCTCTGCTGGATGACG TGAG | 23 | Cpf1 | Exon 2 | 0.9 | |
| 17 | TCAGTGGGGGTGAATTCAG TGTA | 23 | Cpf1 | Exon 2 | 0.5 | |
| 18 | CAGTGGGGGTGAATTCAGT GTAG | 23 | Cpf1 | Exon 2 | 0.4 | |
| 19 | TCACAGCCCAAGATAGTTA AGTG | 23 | Cpf1 | Exon 2 | 0.3 | |

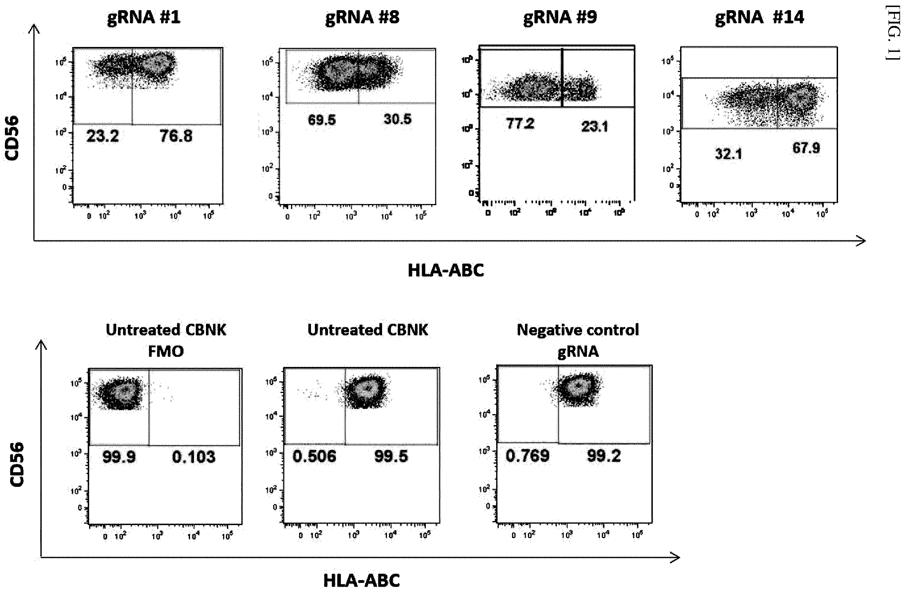

As a result, it was confirmed that the B2M gene was knocked out at the highest rate in 4 gRNA sequences, including #1 (ATCCATCCGACATTGAAGTT), #8 (AGTGGGGGTGAATTCAGTGTA), #9 (AGTGGGGGTGAATTCAGTGTAGT) and #14 (AGCAAGGACTGGTCTTTCTAT), among the 19 sequences (FIG. 1). Among these four sequences, #9 gRNA with the highest efficiency was selected and used to perform B2M gene knockout with Cpf1. When Cpf1 is used, the length of the gRNA sequence is applicable in the range of 19 to 23 bp, and when the same region is targeted, the off-target phenomenon can be minimized as the length of the sequence increases. Meanwhile, in the case of MAD7 nuclease, 21 bp gRNA is used, and thus #8 gRNA, which is 2 bp shorter in length while cleaving the same region as the sequence of #9 gRNA, was used.

The B2M gene in NK cells was cleaved using #8 and #9 gRNAs and Cpf1 or MAD7, and then genomic DNA was isolated and subjected to whole genome sequencing. A genomic DNA sequence having four or less nucleic sequences that mismatch the gRNA sequence was assumed to be a potential off-target region, and whether insertions/deletions by off-target actually occurred was examined. As a result, three predicted off-target regions were identified, and as a result of sequencing for gene insertions/deletions, it was confirmed that no insertions/deletions occurred in two predicted regions. Insertions/deletions were found in the chromosome 4 region, but they were insertions/deletions seen at the same positions as those in control NK. As a result, it was confirmed that B2M knockout using #8 and #9 gRNA did not cause mutation in the predicted off-target regions (Appendix 1).

In this Example, the RNP reaction solution was delivered into the cells using a shuttle (Feldan therapeutics) as a peptide carrier, but various delivery methods such as electroporation, lipofectamine or a cationic polymer (e.g., poly arginine) may also be used.

### Example 2: Evaluation for efficiency of shuttle for B2M gene knockout

Knockout of the B2M gene was performed using a shuttle protein. The sequence of the shuttle used is shown in Table 3 below, and knockout of the B2M gene was performed in the same manner as described in Example 1.

**[Table 3] Sequence of shuttle used**

| Shuttle | Amino acid sequence |
|---|---|
| FSD64d1 | LLKIWSRLIKIWTQGRRLGGSGGGSARAARQAR (SEQ ID NO: 45) |

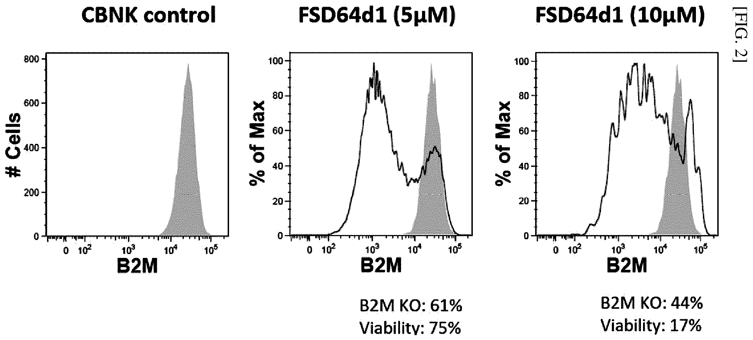

As a result of examining the efficiency of knockout of the B2M gene in CBNK cells using 5 µM and 10 µM of the shuttle, it was confirmed that 5 µM of the FSD64d1 shuttle was superior to 10 µM in terms of B2M knockout efficiency and cell viability (FIG. 2). Thus, in the following Example, B2M knockout was performed using 5 µM of the FSD64d1 shuttle.

### Example 3: Production and in vitro evaluation of low-immunogenic NK cells

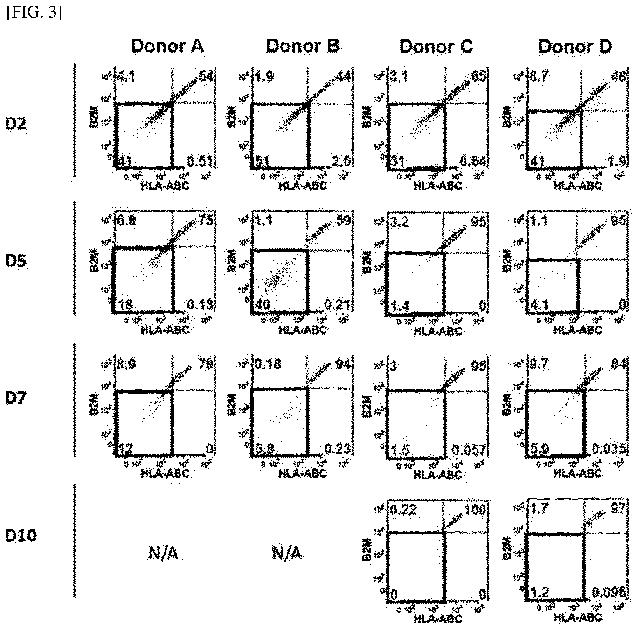

As a result of confirming the expression of B2M and HLA-ABC while culturing B2M gene knockout NK cells, it was confirmed that the proportion of cells, which did not simultaneously express B2M and HLA-ABC, in total cells, decreased with time (FIG. 3). In order to confirm whether the results in FIG. 3 were because NK cells having inhibited expression of HLA-ABC due to B2M gene knockout were attacked by the NK cells expressing HLA-ABC, the following groups were prepared and subjected to *in vitro* apoptosis assay: wild-type CBNK; a group (B2M KO NK) obtained by isolating only *B2M* gene knockout NK cells; a group (HLA-E TD NK) obtained by transducing scHLA-E into wild-type CBNK; a group (B2M KO/HLA-E TD NK) obtained by isolating only *B2M* gene knockout NK cells and transducing scHLA-E thereinto. Since the HLA-E gene is known to inhibit foreign cells from being attacked by NK cells of the host, it was introduced into B2M gene knockout NK cells. It was expected that, when such allogeneic low-immunogenic NK cell having B2M-/HLA-E⁺ expression characteristics were injected *in vivo,* they could evade from being attacked by CD8 (+) T cells and NK cells of the host.

### 3-1) Production of B2M KO NK cells (B2M⁻ NK)

On day 3 of knockout when the proportion of cells not expressing HLA-ABC was maintained at the highest level, B2M gene knockout NK cells were isolated. B2M gene knockout NK cells were collected by centrifugation, and then the supernatant was removed. The cells were resuspended at a concentration of 1×10⁷ cells/100 µL in PBS (Lonza) containing MACS buffer (0.5% FBS (GIBCO), 2 mM EDTA (Invitrogen)), and 10 µg of biotin-conjugated B2M antibody (Invitrogen, MA1-19506) was added per 100 µL of the cell suspension, followed by staining 4°C for 15 minutes. Next, 2 mL of MACS buffer was added thereto, followed by centrifugation at 1,200 rpm for 5 minutes, and the supernatant was removed. Thereafter, the pelleted NK cells were suspended in 80 µL of MACS buffer, and 20 µL of anti-biotin microbeads (Miltenyi Biotec, 130-090-485) were added to the cell suspension and allowed to react at 4°C for 15 minutes. Next, 2 mL of washing buffer (Miltenyi Biotec, 130-091-222) was added to the cell suspension, followed by centrifugation at 1,200 rpm for 10 minutes, and then the supernatant was removed. The NK cells were suspended in 500 µL of washing buffer. An LS column (Miltenyi Biotec) was fixed to QuadroMACS^{™} separator (Miltenyi Biotec) and washed with 2 mL of wash buffer, and the suspended NK cells were passed through the column. Then, B2M knockout cells not bound to the column were recovered by passing 3 mL of washing buffer through the column. After cell counting, the cells were suspended in NK cell medium at a concentration of 1 × 10⁶ cells/ml, and then cultured in a well plate container having an appropriate size.

### 3-2) Production of HLA-E TD NK cells (HLA-E⁺ NK)

A lentiviral vector was used to express a single-chain HLA-E trimer (scHLA-E) in NK cells. As the single-chain HLA-E trimer used in the present invention, the amino acid sequence disclosed in US20050196404A1 was used after codon optimization of the nucleic acid sequence encoding the same (SEQ ID NO: 46). A lentiviral vector expressing the codon-optimized single-chain trimer sequence was produced by Flash Therapeutics (France).

To transduce scHLA-E into NK cells, a complex of 50 MOI (multiplicity of infection) scHLA-E lentiviral vector and 10 µg/mL Protransduzin-A (immundiagnostik/A 2115AG.1) was prepared and left to stand for 5 minutes or more at room temperature, and then the NK cells contained in the culture container were treated with the complex. The medium was co-treated with 6 µM of 5Z-7-oxozeaenol (TOCRIS #360420) and 20 ng/mL of IL-21 (Biolegend/571204) to promote the introduction and expression of the lentiviral vector in the cells.

### 3-3) Production of B2M KO/HLA-E TD NK cells (B2M⁻/HLA-E⁺ NK)

The B2M KO NK cells produced as described in 3-1) above were placed in a culture container, and then transduced with scHLA-E as described in 3-2) above. Finally, low-immunogenic NK cells genetically engineered to have a HLA-ABC⁻/ HLA-E⁺ or HLA-ABC⁻/ HLA-E⁺ were obtained, and the present inventors named the obtained NK cells "Log-NK" (Log-lasting NK). The produced NK cells were analyzed by FACS using the antibodies shown in Table 4 below.

**[Table 4] Antibodies for FACS analysis of NK cell phenotype**

| Marker | Antibody information (fluorescent/ manufacturer/catalog No.) | Amount of antibody used |
|---|---|---|
| Human CD56 | APC / BD Bioscience / 555518 | 5 µL |
| Human HLA-ABC | BV421 / BD Bioscience / 565332 | 0.5 µL |
| Human CD3 | FITC / BD Bioscience / 555332 | 5 µL |
| Human HLA-E | PE / Biolegend / 342604 | 1 µL |
| 7-AAD | PerCp-Cy5.5 / Beckmen Coulter / A07704 | 5 µL |

### Example 4: In vitro characterization of low-immunogenic NK cells

The NK cells of the four groups (wild-type CBNK, B2M⁻ NK, HLA-E⁺ NK, and B2M⁻/HLA-E⁺ NK) to be used as target cells were washed with 1X PBS, and then the supernatant was removed. The cells were suspended at a concentration of 1 × 10⁶ cells/ml in 1 mL of an assay medium, an RPMI 1640 medium (Gibco, 11875093) containing 10% FBS, and then 30 µL of Calcein-AM (Molecular probe, C34852) was added thereto, followed by culture in a CO₂ incubator at 37°C for 1 hour. Next, the cells were washed twice with assay medium and then suspended in 10 mL of assay medium at a concentration of 1 × 10⁵ cells/ml. Meanwhile, PBNK (NK cells from donor peripheral blood) or CBNK (NK cells from donor cord blood) to be used as effector cells were also washed with 1x PBS and then the supernatant was removed, and the cells were suspended in assay medium at a concentration of 3 X 10⁶ cells/ml for an effector: target cell ratio of 30:1 or at a concentration of 1 × 10⁶ cells/ml for an effector: target cell ratio of 10:1. 100 µL of the effector cells prepared at a ratio of 30:1 or 10:1 were added to each of three wells of a round-bottom 96-well plate, and then 100 µL of the target cells at a concentration of 1 × 10⁵/mL were added to each well. Spontaneous release wells and maximum release wells were prepared to calculate the cell killing ability by a formula. 100 µL of stained target cells were added to the span wells, and 100 µL of assay medium was added each. To each spontaneous release well, 100 µL of stained target cells were added and 100 µL of assay medium was added. To each maximum release well, 100 µL of stained target cells were added and 100 µL of 2% Triton-X 100 solution was added. In order to correct the autofluorescence value present in the assay medium and the 2% Triton-X 100 solution, 200 µL of assay medium was added to prepare the medium value, and 100 µL of 2% Triton-X 100 solution was added to 100 µL of assay medium to prepare the value of the mixed solution of the two solutions. The autofluorescence value was corrected by subtracting the value of the mixed solution from the value of the medium to obtain difference (A) and adding difference (A) to the maximum release medium value. The plate was incubated in a CO₂ incubator at 37°C for 4 hours under a light-shielded condition and then centrifuged at 2,000 rpm for 3 minutes. 100 µL of the supernatant was added to each well of a 96-well black plate, and the fluorescence value (OD_{480/535nm}) was measured using a fluorescence plate reader (Perkin Elmer, VICTOR Nivo). Based on the fluorescence value, the ability of the effector cells to kill the target cells was calculated using the following formula:

Cell killing ability (%) = (average fluorescence value of sample wells - average fluorescence value of spontaneous release wells) / {(average fluorescence value of maximum release wells + A) - average fluorescence value of spontaneous release wells } x 100

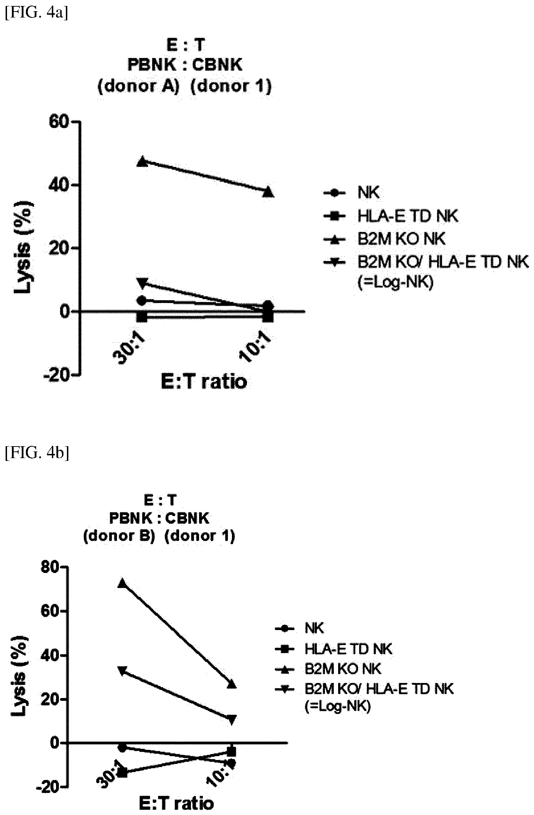

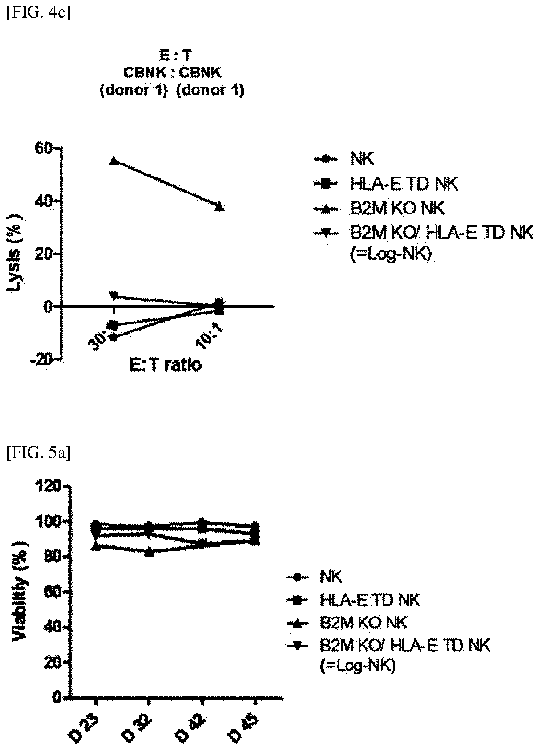

It was confirmed that the B2M knockout (KO) NK group (donor 1) was easily attacked by the PBNK cells derived from another donor (donor A or donor B) or the wild type NK cells from the same donor (donor 1). On the other hand, it was observed that the degree of attack significantly decreased in the HLA-E TD NK or B2M KO/HLA-E TD NK group expressing HLA-E, indicating that the NK cells expressing HLA-E can efficiently evade the attack of NK cells derived from other donors or wild-type NK cells expressing HLA-ABC (FIG. 4). Table 5 below shows the results of measuring the expression of the HLA-E-binding receptors NKG2A and NKG2C on the surfaces of the effector cells by FACS analysis.

**[Table 5] Expression of NKG2A and NKG2C on effector cells**

| Effector cells | NKG2A expression (%) | NKG2C expression (%) |
|---|---|---|
| PBNK (donor A) | 31 | 48 |
| PBNK (donor B) | 76 | 17 |
| CBNK (donor 1) | 96 | 17 |

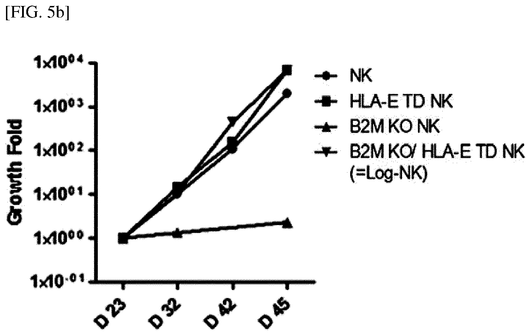

In addition, it was shown that, when the produced genetically engineered NK cell groups were cultured separately, there was no significant difference in viability between the groups, but the growth rate was significantly lower in the B2M KO NK group than in the other groups (FIGS. 5a and 5b).

### Example 5: Optimization of method for producing Log-NK cells (B2M⁻/HLA-E⁺ NK cells)

Log-NK cells having inhibited expression of HLA-ABC due to *B2M* gene knockout and transduced with the HLA-E gene were produced by four different methods as shown in Table 6 below.

**[Table 6] Log-NK production methods**

| | Day 7 | Day 10 | Day 12 | Day 14 | Day 21 | Day 29 | Days 39 to 42 |
|---|---|---|---|---|---|---|---|
| Production Method 1 | B2M KO | B2M-isolation, scHLA-E TD | | | | FACS expression analysis | End of culture (FACS expression analysis) |
| Production Method 2 | | | | scHLA-E TD | B2M KO | FACS expression analysis | End of culture (FACS expression analysis) |
| Production Method 3 | scHLA-E TD | B2M KO | B2M-isolation | | | FACS expression analysis | End of culture (FACS expression analysis) |
| Production Method 4 | B2M KO | | | | | End of culture (FACS expression analysis) | - |
| | | | | | *KO: Knockout, TD: Transduction | | |

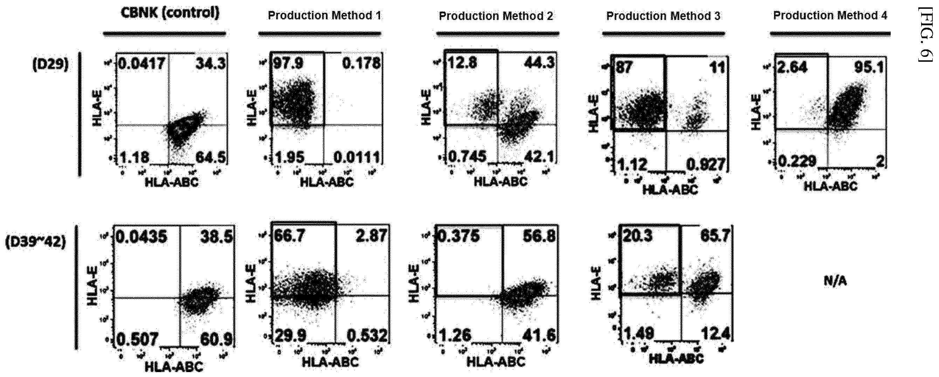

The detailed experimental method of each process and the method for FACS analysis of expression characteristics are as described in Examples 1 and 3. In FIG. 6, the region in the second quadrant (bold solid line) of each graph represents Log-NK cells. In the case of Production Method 1, on day 7 which is the initial time point of NK cell culture, cells were treated with B2M gRNA, and after 3 days, only B2M- cells were isolated, and then scHLA-E was transduced into the cells and expressed. As a result, it was confirmed that 96% or more of Log-NK cells were present until the end of culture.

In the case of Production Method 2, on day 14 corresponding to 1/3 of the entire culture period, scHLA-E was transduced into cells and expressed, and after 3 days, B2M was knocked out, and the process of isolating B2M⁻ cells was not performed. Since cells expressing HLA-E can evade the attack of other NK cells, it was expected that, even if the B2M gene was knocked out, the expression of HLA-E would continue and the survival and growth of the cells could be maintained. However, the actual results showed that the Log-NK cell production efficiency was low (about 13%) and the Log-NK cells present decreased over the culture time, and thus there were no Log-NK cells remaining at the end point.

In the case of Production Method 3, the production process was started on day 7 of culture as in Production Method 1, and B2M knockout and HLA-E transduction were performed in reverse order, followed by the process of isolating B2M- cells, thereby producing Log-NK cells. As a result, it was confirmed that the Log-NK cell production efficiency (87%) was higher than that in preparation method 2, but the Log-NK cells were reduced to about 20% at the end of culture, resulting in a very low yield.

In the case of Production Method 4, B2M was knocked on day 7 of culture, and HLA-E was transduced without the process of isolating B2M- cells, followed by culturing. It was confirmed that Log-NK cells were hardly obtained (about 3%) compared to the previous three production methods.

From the above results, it was confirmed that Log-NK cells of the present invention, which inhibit B2M gene expression for elimination of immunogenicity by inhibition of HLA-ABC expression and express HLA-E to evade the attack of other NK cells, were best obtained by Production Method 1.

### Example 6: Culture and characterization of NK or Log-NK cells

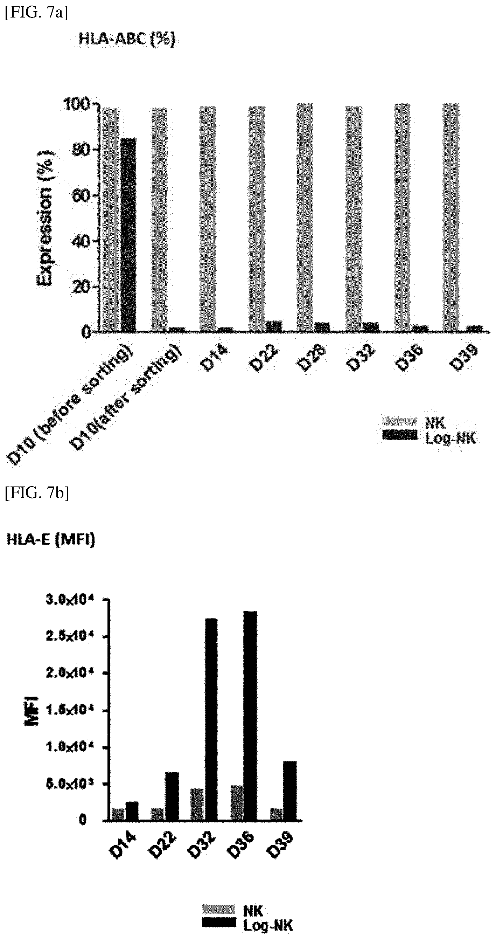

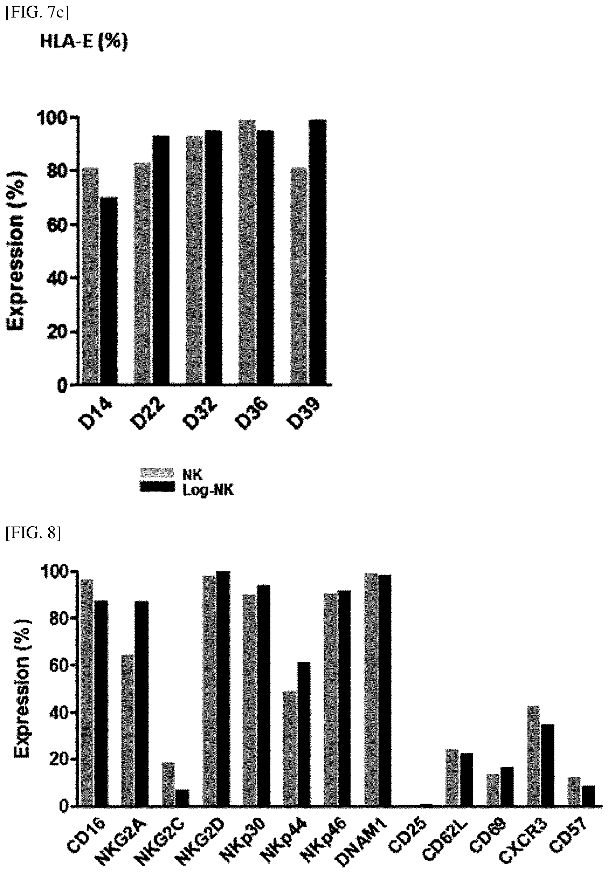

CBNK or Log-NK cells were cultured with restimulation using feeder cells on the start date of culture, day 14 of culture, and day 28 of culture. The feeder cells used in culture were CD4+ T cells transformed with 4-1BBL, mbIL-21 (membrane bound IL-21) and mTNF-α (membrane TNF-α) genes. Restimulation using feeder cells is possible at intervals ranging from 14 to 16 days. Log-NK cells that were stationary cultured in an appropriate culture container were frozen after up to 39 days of culture, and could be cultured for up to 42 days depending on the cell growth rate. If more than 42 days of culture is required, continuous culture is possible by restimulation with feeder cells on day 42 of culture. NK or Log-NK cells were stained with the antibodies shown in Table 7, and whether the Log-NK (HLA-ABC-/HLA-E⁺) phenotype was maintained in NK cells with CD3⁻/CD56⁺ purity was checked up to the end of culture. As a result, it was confirmed that the expression of HLA-ABC gene in the Log-NK cells was maintained at 5% or less, and the HLA-E expression level (mean fluorescence intensity, MFI) in each type of cells was maintained higher than that in the wild-type NK cells (FIG. 7). At the end of the culture, to identify NK cell-specific markers, antibodies corresponding to markers listed in Table 7 together with the human CD56, human CD3 and 7AAD antibodies shown in Table 4 were used to make a total of 14 staining tubes, and then expression of the markers was analyzed by flow cytometry in the same manner as described in Example 3. As a result, it was confirmed that there was no significant decrease in activity markers or no significant increase in inhibitory markers compared to those in the wild-type NK cells (FIG. 8).

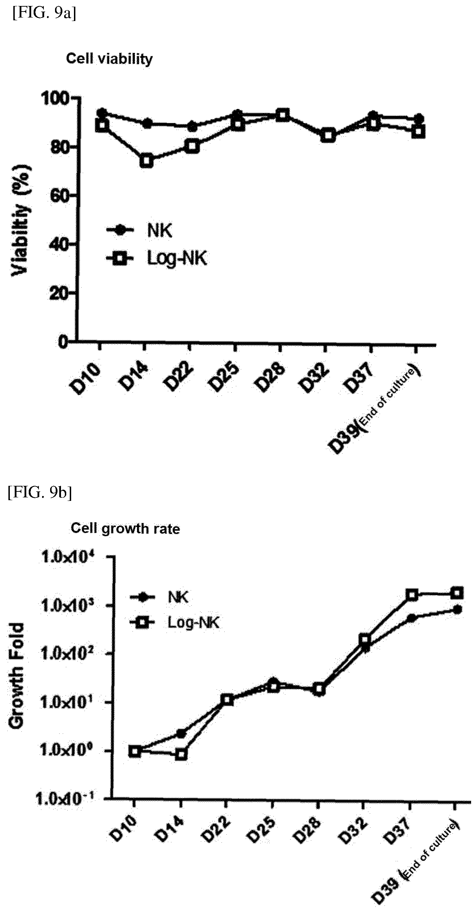

The Log-NK cells produced by Production Method 1 showed a similar cell viability compared to the wild-type NK cells during a period ranging from day 10 of culture (corresponding to the end of the production process) to day 39 corresponding to the end of culture. The decreases in the viability and growth rate of Log-NK cells, which appeared between day 10 and day 14 of culture, were a phenomenon that appeared temporarily due to the death of the B2M-NK cells remaining untransformed with the HLA-E lentiviral vector, and it could be confirmed that, after restimulation with the feeder cells on day 14, both the growth rate and the viability were restored (FIG. 9).

**[Table 7] List of antibodies for FACS analysis of NK characteristics**

| Marker (human) | Antibody information (fluorescent/ manufacturer/catalog No.) | Amount of antibody used |
|---|---|---|
| Human CD16 | PE / BD Bioscience / 555407 | 5 µL |
| Human NKG2A | PE / Miltenyi Biotec / 130-113-566 | 1 µL |
| Human NKG2C | PE / R&D Systems / FAB138P | 1 µL |
| Human NKG2D | PE / BD Bioscience / 557940 | 1 µL |
| Human NKp30 | PE / BD Bioscience / 557940 | 1 µL |
| Human NKp44 | PE / BD Bioscience / 558563 | 5 µL |
| Human NKp46 | PE / BD Bioscience / 557991 | 5 µL |
| Human DNAM1 | PE / BD Bioscience / 559789 | 5 µL |
| Human CD25 | PE / BD Bioscience / 555432 | 5 µL |
| Human CD62L | PE /Invitrogen / 12-0629-42 | 1 µL |
| Human CD69 | PE / R&D Systems / FAB23591P | 1 µL |
| Human CXCR3 | PE / BD Bioscience / 557185 | 5 µL |
| Human CD57 | PE / BD Bioscience / 560844 | 1 µL |
| Mouse IgG1k | PE / BD Bioscience / 555749 | 5 µL |

### Example 7: Assessment of tumor cytotoxicity of Log-NK cells

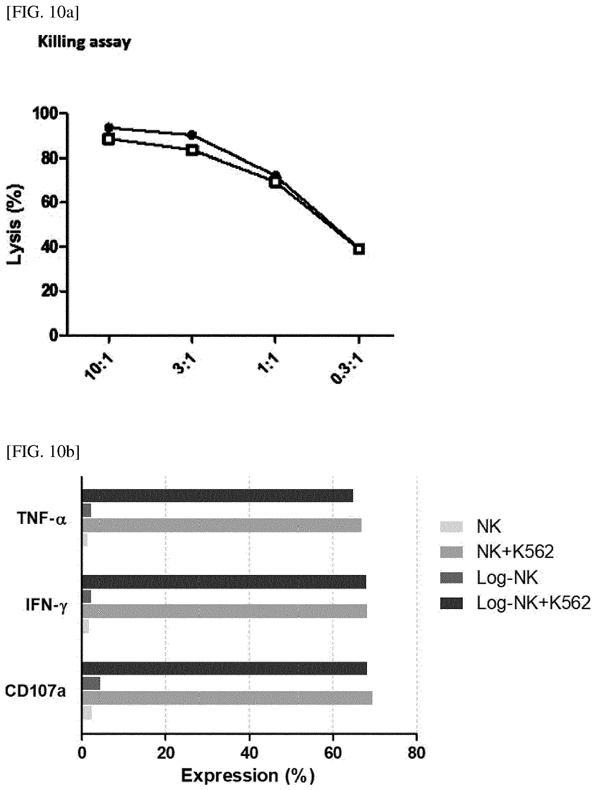

To evaluate the tumor-killing ability of wild-type NK cells or Log-NK cells, the K562 hematological cancer cell line was used as target cells. K562 cells were washed with 1X PBS, and then the supernatant was removed. The cells were suspended at a concentration of 1 × 10⁶ cells/ml in an assay medium, which is an RPMI 1640 medium containing 10% FBS, and then 30 µL of Calcein-AM (Molecular probe, C34852) was added thereto, followed by culture in a CO₂ incubator at 37°C for 1 hour. Next, the cells were washed twice with 10 mL of assay medium and then suspended in 10 mL of assay medium at a concentration of 1 × 10⁵ cells/ml. Wild-type NK cells or Log-NK cells were washed with 1x PBS, and then the supernatant was removed, the cells were suspended in assay medium at a concentration of 1 × 10⁶ cells/ml for an effector: target cell ratio of 10:1, a concentration of 3 × 10⁵ cells/ml for an effector: target cell ratio of 3:1, a concentration of 1 × 10⁵ cells/ml for an effector: target cell ratio of 1:1, or a concentration of 3 × 10⁴ cells /ml for an effector: target cell ratio of 0.3:1. 100 µL of the effector cells prepared at four ratios were added to each of three wells of a round-bottom 96-well plate, and then 100 µL of the target cells at a concentration of 1 × 10⁵/mL were added to each well. Subsequent experimental procedures and the calculation of tumor cell killing ability were performed in the same manner as in Example 4. As a result of evaluating the ability to kill the K562 cancer cell line, it was confirmed that there was no significant difference in cancer cell killing ability between NK cells and Log-NK cells at a high E:T ratio, and as the E:T ratio decreased, the difference in cancer cell killing ability between the NK cells and the Log-NK cells completely disappeared (FIG. 10a).

### Example 8: Examination of cytokine secretion by Log-NK cells

The secretion amounts of CD107a, IFN-γ and TNF-α, which are major cytokines secreted by NK cells to kill cancer cells, were examined by an intracellular cytokine staining (ICS) assay method. Antibodies used in the ICS experiment are specified in Table 8 below, and the types of antibodies used in each cell staining method are specified in Table 9 below.

**[Table 8] List of antibodies for ICS**

| Antibody | Antibody information (fluorescent/ manufacturer/catalog No.) | Amount of antibody used |
|---|---|---|
| Human IFN-γ | FITC / BD Bioscience / 554700 | 1 µL |
| Human TNF-α | PE-CY7 / eBioscience / 25-7349-82 | 1 µL |
| Human CD3 | PerCP-Cy5.5 / eBioscience / 45-0036-42 | 5 µL |
| Human CD56 | APC-Cy6 / eBioscience / 47-0567-42 | 1 µL |
| Human CD107a | APC / BD Bioscience / 560664 | 1 µL |
| 7-AAD | PerCp-Cy5.5 / Beckmen Coulter / A07704 | 5 µL |
| Mouse IgG1k | FITC / BD Bioscience / 555748 | 5 µL |
| Mouse IgG1k | PE-Cy7 / BD Bioscience / 557872 | 5 µL |
| Mouse IgG1k | APC / BD Bioscience / 555751 | 5 µL |

**[Table 9] List of antibodies for each cell staining method**

| | Antibody | | |
|---|---|---|---|
| | Fluorescent | (-), target well | iso well |
| Surface staining | PerCP-cy5.5 | 7AAD/CD3 | |
| | APC-Cy7 | CD56 | |
| | APC | CD107a | IgG1k |
| Intracellular staining | FITC | IFN-γ | IgG1k |
| | PE-Cy7 | TNF-α | IgG1k |

Wild-type NK cells or Log-NK cells as effector cells were washed with 1x PBS, and then supernatant was removed. The cells were suspended in an assay medium, which is 10% FBS-containing RPMI 1640 medium, at a concentration of 2.5×10⁶ cells/mL for an effector: target cell ratio of 1:1. Next, 1.2 mL of the suspension was placed in a fresh tube, and 1.56 µL of Golgi-stop (BD, 554724) and 2.4 µL of Golgi-plug (BD, 555029) were added thereto.

Meanwhile, K562 cells as target cells were suspended in assay medium at a concentration of 2.5 x 10⁶ cells/ml. A 96-well round bottom plate was prepared, APC-CD107a antibody was added to (-) and target wells, and APC-IgG1k antibody was added to iso wells. To each (-) well, 100 µL of 10% FBS-containing RPMI 1640 medium was added, and to each of the target and iso wells, 100 µL of the target cells were added. 100 µL of the effector cells were added to each of the wells and then incubated in a CO₂ incubator at 37°C for 4 hours. The plate was centrifuged at 2,000 rpm for 3 min at 4°C and washed twice with 200 µL of perm/wash buffer (BD, 554723), and then 200 µL of fixation/permeabilization buffer (BD, 554655) was added to each well, followed by incubation at 4°C for 30 minutes. The plate was centrifuged at 2,000 rpm for 3 min at 4°C and washed twice with 200 µL of perm/wash buffer (BD, 554723), and then 100 µL of perm/wash buffer (BD, 554723) was added to each well. To the (-) and target wells, IFN-γ and TNF-α antibodies were added, and to the iso wells, FITC-IgG1k and PE-Cy7-IgGlk antibodies were added, followed by incubation at 4°C for 30 minutes. 100 µL of 1X perm/wash buffer was added to each well, and the plate was centrifuged at 2,000 rpm for 3 min at 4°C, and the supernatant was removed. The plate was centrifuged at 2,000 rpm for 3 min at 4°C and washed twice with 200 µL of perm/wash buffer (BD, 554723), and then 200 µL of 1X perm/wash buffer was added to each well. The cell pellet was transferred to a FACS tube (BD falcon, 352052) and the cytokine expression therein was analyzed by flow cytometry. As a result, it was confirmed that, when the NK cells and the Log-NK cells were in contact with the K562 target cells, the major cytokines CD107a, IFN-γ and TNF-α in the NK cells and Log-NK cells all increased by more than 60%, and there was no significant difference in the cytokines between the NK cell group and the Log-NK cell group (FIG. 10b).

### Example 9: In vitro evaluation of low immunogenicity of Log-NK cells

An *in vitro* mixed lymphocyte reaction (MLR) assay was performed to evaluate the *in vivo* low-immunogenicity of the produced Log-NK cells. Using a CD8 (+) T cell isolation kit (Miltenyi Biotec, 130-096-495), CD8 (+) T cells were isolated as follows. PBMCs obtained from donor blood were washed once with PBS (Lonza) containing MACS buffer 0.5% FBS (GIBCO) and 2 mM EDTA (Invitrogen), and then the obtained cell pellet was suspended in MACS buffer to a cell concentration of 1 × 10⁷ cells/10 µL. Biotin-antibody cocktail was added to the suspension to reach a concentration of 1 × 10⁷ cells/10 µL, mixed well, and incubated at 4°C for 5 minutes. After completion of the reaction, MACS buffer was added to reach a cell concentration of 1 × 10⁷ cells/30 µL, and 20 µL of CD8(+) T cell microbead cocktail was added per 1 × 10⁷ cells, followed by incubation at 4°C for 10 minutes. After completion of incubation, the cell suspension was passed through an LS column (Miltenyi Biotec) to isolate only T cells expressing CD8.

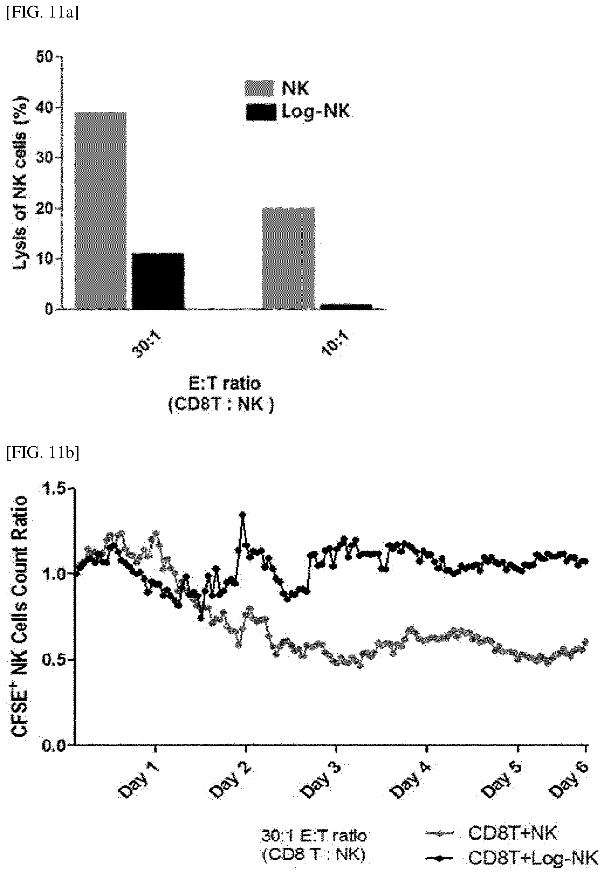

In order to culture the isolated CD8(+) T cells, PBMCs donated from another donor were irradiated with 2,000 cGy and then mixed with the CD8(+) T cells at a 1:1 ratio, followed by culture for 7 days. PBMCs were prepared in the same manner and mixed at a ratio of 1:1 with the CD4(+) T cells and CD8(+) T cells cultured for 7 days. The cells were cultured in RPMI 1640 medium containing anti-CD3 (1 µg/mL, Invitrogen), IL-2 (100 U/mL) and 10% BBS for 7 days. The cells were further cultured for 7 days (a total of 14 days of culture) and used in the experiment. The same donor-derived PBMCs used above were irradiated again with 2,000 cGy, mixed with the cultured CD8(+) T cells at a ratio of 1:1, and further cultured for one week. Using the CD8(+) T cells cultured for a total of 14 days as effector cells, and using NK or Log-NK cells as target cells, whether Log-NK cells could evade the attack of CD8(+) T cells was examined by measuring cell death in the same manner as in Example 3. As shown in FIG. 11a, it was confirmed that the proportion of cells lysed by CD8(+) T cells was 3 times higher in NK cells than in Log-NK cells, indicating that Log-NK could evade the attack of CD8(+) T cells. In order to facilitate the MLR reaction, the experiment was conducted under conditions where the HLA-A, B, and C types of CD8(+) T cells 100% mismatched with those of and NK or Log-NK cells and where the irradiated PBMCs used for culture of CD8(+) T cells were 83% identical with the HLA-A, B, and C types of NK or Log-NK cells.

In addition, NK and Log-NK cells were stained with CFSE (Thermo scientific, C34554), and then 100 µL of CD8(+) T cells at a concentration of 1.2×10⁶ cells /mL in RPMI 1640 (10% FBS, 1000 IU IL-2) medium and 100 µL of the NK or Log-NK cells at a concentration of 4×10⁴ cells /mL in the medium were added to each of 3 wells of a 96-flat-well plate so that the ratio of CD8(+) T : NK or Log-NK was 30:1. The plate was analyzed using the IncuCyte^{®}S3 system (Sartorius) provided inside the cell culture incubator, well images were stored every hour in a live state, and then only cells positive for CFSE fluorescence on the images was counted. As a result, it could be confirmed that, when NK cells were co-cultured with CD8(+) T cells, the ratio of the cultured NK cells to the NK cells initially added to the wells decreased over time, whereas, in the case of Log-NK cells, the number of the cells initially added to the wells was maintained (FIG. 11b).

### Example 10: Selection of optimal gRNA sequence for CIITA gene knockout

### 10-1) Knockout of CIITA and RFXANK genes using gene scissors

Human major histocompatitibility complex (MHC) class II genes include HLA-DR, HLA-DQ, and HLA-DP. In order to produce NK cells that do not express HLA-DR/DQ/DP, a desired portion of Class II transactivator (CIITA) known as a master controller or the transcription factor RFXANK (RFX-associated ankyrin-containing protein gene was delivered in the following manner using gene scissor technology to inhibit the expression of CIITA or RFXANK.

Each of the CIITA- or RFXANK-targeting gRNA candidate sequences listed in Table 10 below was prepared using an RNP reaction solution in the same manner as in Example 1, and the reaction solution was used within 60 minutes after reaction at room temperature for 5 minutes or more.

The prepared RNP reaction solution could be used for up to 1 to 4 x 10⁶ cells, and NK cells were counted using an ADAM cell counter system (NanoEntek) and then 2 X 10⁶ NK cells were used. Subsequent experimental procedures and cell culture were performed in the same manner as in Example 1.

**[Table 10] gRNA candidate sequence targeting CIITA or RFXANK**

| No. | SEQID NO. | Nuclease | gRNA -exon position | Sequence | Length (bp) | NK cell culture period after knockout (KO % of HLA-DR) | | | |
|---|---|---|---|---|---|---|---|---|---|
| 1 | 20 | Cpf 1 | CIITA-ex1A | CCTTGGGGCTCTGAC AGGTAGGA | 23 | D4 (29.7) | D8 (28.2) | | |
| 2 | 21 | Cpf 1 | CIITA-ex1B | CCGGCCTTTTTACCTT GGGGCTC | 23 | D4 (-) | D8 (7.3) | | |
| 3 | 22 | Cpf 1 | CIITA-ex3A | CTCCCAGAACCCGAC ACAGACAC | 23 | D4 (9.6) | D8 (12.4) | | |
| 4 | 23 | Cpf 1 | CIITA-ex4A | CTTGTCTGGGCAGCG GAACTGGA | 23 | D4 (4.6) | D8 (5.3) | | |
| 5 | 24 | Cpf 1 | CIITA-ex5A | TGCCCAACTTCTGCTG GCATCTC | 23 | D5 (5.6) | D7 (11.4) | D10 (14.9) | D14 (17.7) |
| 6 | 25 | Cpf 1 | CIITA-ex5B | TGACTTTTCTGCCCAA CTTCTGC | 23 | D5 (0.6) | | | |
| 7 | 26 | Cpf 1 | CIITA-ex7A | TCCAGGCGCATCTGG CCGGAGGC | 23 | D5 (2.0) | | | |
| 8 | 27 | Cpf 1 | CIITA-ex8A | TCCCCACCATCTCCAC TCTGCCC | 23 | D5 (5.4) | D7 (13.1) | D10 (14.9) | D14 (19.9) |
| 9 | 28 | Cpf 1 | CIITA-ex8B | TCCAGTTCCTCGTTGA GCTGCCT | 23 | D5 (-) | | | |
| 10 | 29 | Cpf 1 | CIITA-ex8C | CCAGAGCCCATGGGG CAGAGTGG | 23 | D5 (5.5) | D8 (10.4) | | |
| 11 | 30 | Cpf 1 | CIITA-ex9A | CTCGGGAGGTCAGGG CAGGTTCA | 23 | D5 (8.9) | D8 (11.2) | | |
| 12 | 31 | Cpf 1 | CIITA-ex10A | GAAGCTTGTTGGAGA CCTCTCCA | 23 | D5 (-) | | | |
| 13 | 32 | Cpf 1 | CIITA-ex11A | GCAGCACGTGGTACA GGAGCTCC | 23 | D5 (3.5) | D8 (9.1) | | |
| 14 | 33 | Cpf 1 | CIITA-ex11B | GCCATCGCCCAGGTC CTCACGTC | 23 | D5 (5.1) | | | |
| 15 | 34 | Cpf 1 | CIITA-ex11C | AGAGCTCAGGGATGA CAGAGCAC | 23 | D5 (-) | | | |
| 16 | 35 | Cpf 1 | RFXANK-ex1A | CAGTAGGAGTAGTCG TCGCGTAT | 23 | D5 (-) | | | |
| 17 | 36 | Cpf 1 | RFXANK-ex1B | CAGGGCAAGGGTGCG CAGGCGCA | 23 | D5 (-) | | | |
| 18 | 37 | Cpf 1 | RFXANK-ex1C | CGAGATCTACTGAAC CAGAAAAG | 23 | D5 (-) | | | |
| 19 | 38 | Cpf 1 | RFXANK-ex1D | TGGTTCAGTAGATCTC GTAAAGA | 23 | D5 (-) | | | |
| 20 | 39 | Cpf 1 | RFXANK-ex2A | GGACAAGGTTCTCTG CAACCTGA | 23 | D5 (-) | | | |
| 21 | 40 | MAD7 | CIITA-ex1A | CCTTGGGGCTCTGAC AGGTAG | 21 | D5 (22.6) | D7 (15.5) | | |
| 22 | 41 | MAD7 | CIITA-ex1B | GGGCTCTGACAGGTA GGACCC | 21 | D4 (1.0) | D8 (0) | | |
| 23 | 42 | MAD7 | CIITA-ex3A | CCTCCCAGGCAGCTC ACAGTG | 21 | D4 (1.3) | D8 (0) | | |
| 24 | 43 | MAD7 | CIITA-ex3B | TATGACCAGATGGAC CTGGCT | 21 | D4 (0.3) | D8 (0) | | |
| 25 | 44 | MAD7 | CIITA-ex5A | TGCCCAACTTCTGCTG GCATC | 21 | D5 (38.6) | D7 (40.1) | | |

### 10-2) NK cell staining for flow cytometry

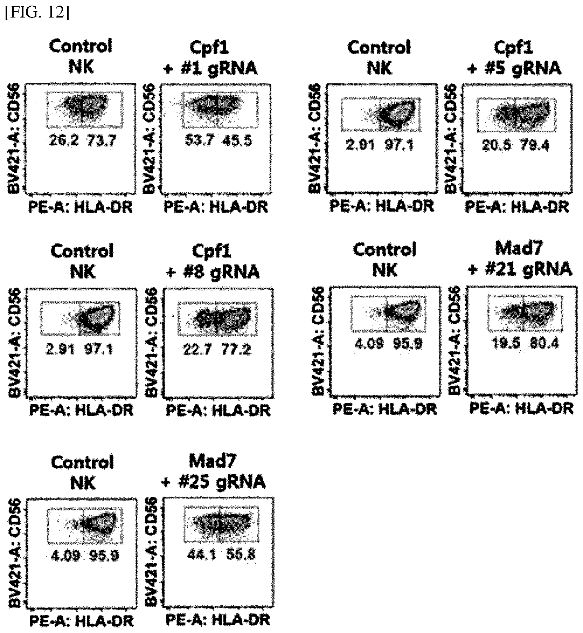

In order to examine the degree of knockout of CIITA or RFXANK gene in NK cells, NK cells treated with the CIITA RNP reaction solution in Example 10-1) were stained and then analyzed by flow cytometry. 2 x 10⁵ NK cells were suspended in 2 mL of 2% FBS-containing FACS buffer and centrifuged at 2,000 rpm for 3 minutes. Then, the cells were in 100 µL FACS buffer, and fluorescently labeled antibodies (Table 11) were added thereto, followed by incubation at 4°C for 30 min. After 30 min, 2 mL of FACS buffer was added to the cells, followed by centrifugation at 2,000 rpm for 3 minutes. Next, 300 µL of a fixation solution was added to the cells which were then analyzed using the flow cytometer BD LSRFortessa (BD Bioscience), and the results are shown in FIG. 12.

**[Table 11] List for antibodie for FACS analysis**

| Marker | Antibody information (fluorescent/ manufacturer/Catalog No.) | Amount of antibody used |
|---|---|---|
| Human B2M | APC / BD Bioscience / 316312 | 0.5 µL |
| Human HLA-DR | PE / Invitrogen / 12-9956-42 | 1 µL |
| Human CD56 | BV421 / BD Bioscience / 562751 | 0.5 µL |
| Human CD3 | FITC / BD Bioscience / 555332 | 5 µL |
| 7-AAD | PerCp-Cy5.5 / Beckmen Coulter / A07704 | 5 µL |

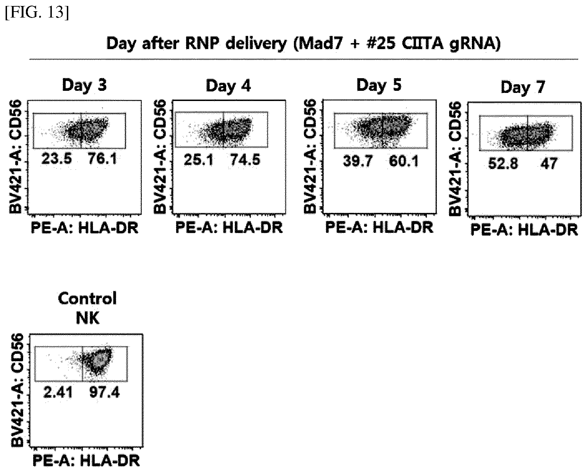

The degree of knockout of HLA class II was examined based on by the expression of HLA-DR. It was confirmed that NK cells treated with Cpf1-#1 gRNA among a total of 25 gRNA sequences tested showed a CIITA gene knockout efficiency of 28.2% compared to the control wild-type NK cells, and NK cells treated with Cpf1-#5 gRNA showed a knockout efficiency of 17.7%, and NK cells treated with Cpf1-#8 gRNA showed a knockout efficiency of 19.9%. In addition, NK cells treated with MAD7-#21 gRNA was a knockout efficiency of 15.5%, and NK cells treated with MAD7-#25 gRNA showed a knockout efficiency of 40.1%. Therefore, MAD7 nuclease and #25 gRNA showing the best CIITA gene knockout efficiency were selected and used in subsequent experiments. In addition, as shown in FIG. 13, as a result of examining the CIITA knockout efficiency on days 3 to 7 days of culture after delivery of the RNP reaction solution, it was confirmed that the CIITA knockout efficiency increased from 21.3% on day 3 of culture to 50.4% on day 7 of culture compared to the control NK cells. These results show that the knockout efficiency increases as the culture period after CIITA knockout in NK cells increases.

### Example 11: Optimization of conditions for production of NK cells knocked-out of B2M and CIITA genes

To produce NK cells knocked-out of B2M and CIITA genes, the NK cells were produced by two methods as shown in FIG. 14a, and the efficiency of double knockout of the B2M and CIITA genes was compared between the two methods. In Production Method 1, in a 1.5-mL centrifugation tube, 13 µL of 1X PBS, 2 µL of 3.2 µM MAD7 and 10 µL of 10 µM B2M gRNA were mixed together and then incubated at room temperature for 5 minutes or more, thus preparing RNP reaction solution tube 1. In another 1.5-mL centrifugation tube, 13 µL of 1X PBS, 2 µL of 3.2 µM MAD7 and 10 µL of 10 µM CIITA gRNA were incubated at room temperature for 5 minutes or more, thus preparing RNP reaction solution tube 2. The prepared RNP reaction solution tubes 1 and 2 were mixed together, thus preparing a total of 50 µL of RNP reaction solution. Subsequent experimental procedures were performed on 2 × 10⁶ NK cells (on day 7 of culture) in the same manner as in Example 10. In Production Method 2, CIITA in 2 x 10⁶ NK cells on day 7 of culture was knocked out, followed by culture for 4 days, and then B2M in 2 x 10⁶ NK cells on day 11 of culture was knocked out. Specific experimental procedures and cell culture were performed in the same manner as in Example 1. The NK cells cultured after knockout were stained with antibodies for FACS in the same manner as in Example 10-2), and then efficiency of knockout of the B2M and CIITA genes was examined by flow cytometry.

As can be seen in FIG. 14b, as a result of conducting the experiment using the above-described production methods on the NK cells isolated from three donors, it was confirmed that the double knockout efficiency of B2M and CIITA in the NK cells produced from donor 1 by Production Method 1 was 32.4% compared to the control NK cells, and the double knockout efficiency in the NK cells produced by Production Method 2 was 17%. For the NK cells isolated from the other two donors, Production Method 1 showed higher double knockout efficiency than Production Method 2.

Genomic DNA was isolated from the B2M- and CIITA-double knockout NK cells produced by Production Method 1, and then subjected to whole genome sequencing. A genomic DNA sequence having four or less nucleic sequences that mismatch the gRNA sequence of each of B2M and CIITA was assumed to be a potential off-target region, and whether insertions/deletions by off-target actually occurred was examined. As a result, three predicted off-target regions were identified in the B2M gRNA sequence (FIG. 14c), and two predicted off-target regions were identified in the CIITA gRNA sequence (FIG. 14f). As a result of sequencing for gene insertions/deletions, it was confirmed that no insertions/deletions occurred in any of the predicted off-target regions, and as a result, it was confirmed that the double knockout of B2M and CIITA using #8 gRNA (SEQ ID NO: 8) and #25 gRNA (SEQ ID NO: 44) caused no mutations in the predicted off-target regions (FIGS. 14d, 14e, 14g and 14h).

### Example 12: Production of Log-NK-CIITA KO cells (B2M-/CIITA-/HLA-E+ NK cells)

NK cells having an HLA-ABC⁻/HLA-DR/DP/DQ⁻/HLA-E⁺ phenotype as a result of inhibiting the expression of the type II HLA protein HLA-DR/DP/DQ by knocking out the CIITA gene in Log-NK cells having B2M-/HLA-E+ expression characteristics were produced by Production Method 1 or Production Method 2 shown in FIG. 14a, and the produced NK cells were named "Log-NK-CIITA KO" cells. It was expected that, when allogeneic low-immunogenic NK cells having inhibited expression of both type I HLA and type II HLA were injected *in vivo,* they could evade immune responses caused by CD8(+) T cells, CD4(+) T cells and NK cells in the host. NK cells knocked-out of B2M and CIITA were produced in the same manner as in Production Method 1 of Example 11, and then NK cells not expressing B2M were isolated using an MACS (Magnetic Activated Cell Sorting) system, and then cells not expressing HLA-DR were isolated. The cells were incubated with biotin-labeled B2M antibody (Invitrogen) at 4°C for 20 minutes, and then 2 mL of MACS buffer (0.5% FBS, 2 mM EDTA in PBS) was added thereto, followed by centrifugation at 1,200 rpm for 10 minutes. After removal of the supernatant, 80 µL of MACS buffer was added to the cells, and 20 µL of anti-biotin microbeads (Miltenyi Biotec) were added thereto, followed by incubation at 4°C for 15 minutes. Next, 2 mL of MACS buffer was added to the cells, followed by centrifugation at 1,200 rpm for 10 min. The NK cells were suspended in 500 µL of washing buffer, and then the cells were passed through the LS column (Miltenyi Biotec) attached to QuadroMACS^{™} separator (Miltenyi Biotec). Then, 3 mL of washing buffer was passed twice through the column to recover B2M⁻ cells not bound to the column. The recovered cells were centrifuged again at 1,200 rpm for 10 minutes, the supernatant was removed. Next, 80 µL of washing buffer was added to the pelleted cells, 20 µL of anti-HLA-DR microbeads (Miltenyi Biotec) were added thereto, followed by incubation at 4°C for 15 minutes, and 2 mL of washing buffer was added to the cells, followed by centrifugation at 1,200 rpm for 10 minutes. The NK cells were suspended in 500 µL of buffer, and then passed through the LD column (Miltenyi Biotec) attached to QuadroMACS^{™} separator (Miltenyi Biotec), and 1 mL of washing buffer was passed twice through the column to finally recover B2M⁻/HLA-DR⁻ NK cells. The isolated B2M-/HLA-DR- NK cells were counted, and then immediately, a single-chain HLA-E trimer (scHLA-E) was introduced into the cells using a lentiviral vector in the same manner as in Example 3-2). The NK cells were suspended in a medium at a concentration of 1 × 10⁶ cells/mL and cultured in a well plate having an appropriate size.

### Example 13: Culture of Log-NK-CIITA KO cells and confirmation of phenotypic expression

### 13-1) Measurement of expansion ability of Log-NK-CIITA KO cells

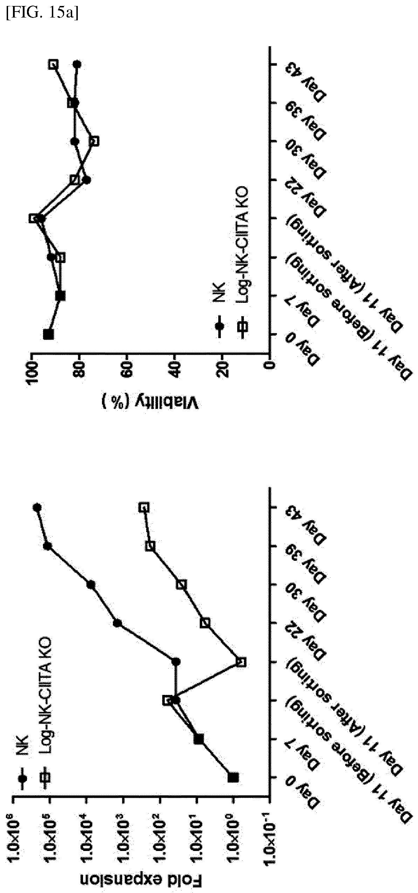

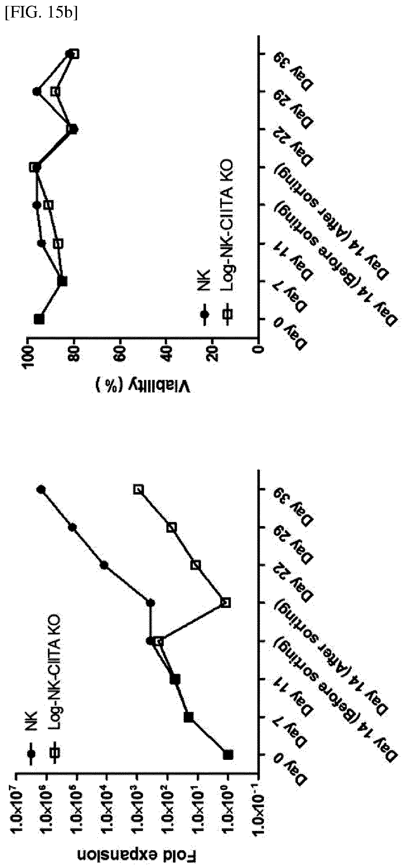

As shown in FIG. 15, in order to confirm the expansion ability of the Log-NK-CIITA KO cells produced in Example 12, cell expansion and cell viability were measured during the culture period. As a result, it was confirmed that the viability of the Log-NK-CIITA KO cells produced by Production Method 1 did not significantly differ from the viability of the control NK cells, and the expansion of the control NK cells continued to increase for 43 days of culture and the number of the Log-NK-CIITA KO cells on day 43 of culture increased about 268-fold compared to the number of the cells at the start of culture (FIG. 15a). It was confirmed that the number of the Log-NK-CIITA KO cells produced by production method 2 decreased after the cell isolation process of Example 12, but cell expansion was restored until day 39 of culture, and the number of the cells day 39 of culture increased about 936-fold compared to the number of the cells at the start of culture (FIG. 15b).

### 13-2) Analysis of phenotype of Log-NK-CIITA KO cells during culture period

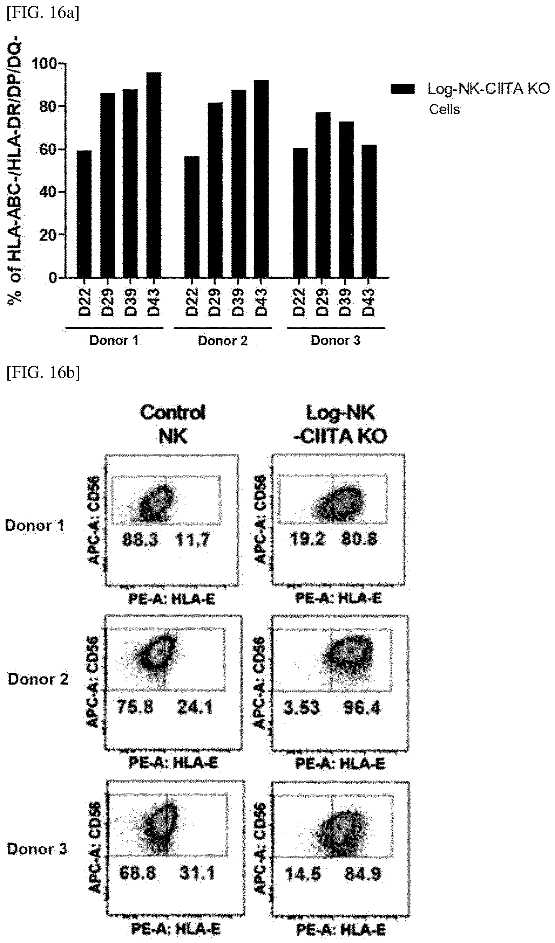

In order to examine whether the expression of HLA-ABC and HLA-DR/DP/DQ was continuously inhibited for 43 days of culture after the production of Log-NK-CIITA KO cells, the NK cells produced by the method of Example 11 were stained, and then the expression in the cells was analyzed by flow cytometry. 2 mL of FACS buffer containing 2% FBS was added to 2 x 10⁵ NK cells, followed by centrifugation at 2,000 rpm for 3 minutes, and then the supernatant was removed. The cells were suspended in 100 µL of FACS buffer and incubated with the antibodies listed in Table 12 at 4°C for 30 minutes. Next, 2 mL of FACS buffer was added to the cells, followed by centrifugation at 2,000 rpm for 3 minutes, and the supernatant was removed. Next, 300 µL of fixative solution was added to the cells, and then expression was analyzed by the flow cytometer BD LSRFortessa (BD Bioscience). As a result, as shown in FIG. 16a, it was confirmed that, in the case of all three donors, the cells having inhibited expression of both HLA-ABC and HLA-DR/DP/DQ were 60% or more of the total cells after 22 days of culture, and in the case of donors 1 and 2, the number of the cells increased to more than 90% of the total cells on day 43 of culture. In addition, as a result of analyzing the expression of HLA-E in the Log-NK-CIITA KO cells on day 43 of culture, it was confirmed that the expression of HLA-E increased by 69.1% in donor 1, 72.3% in donor 2, and 53.8% in donor 3 compared to the control NK cells (FIG. 16b).

**Table 12 List of antibodies for FACS analysis**

| **Marker** | **Antibody information (fluorescent/manufacturer/catalog No.)** | **Amount of antibody used** |
|---|---|---|
| Human HLA-DR/DP/DQ | FITC / BD Bioscience / 555558 | 5 µL |
| Human CD56 | APC / BD Bioscience / 555518 | 5 µL |
| Human HLA-ABC | BV421 / BD Bioscience / 565332 | 0.5 µL |
| Human CD3 | PE-Cy7 / Invitrogen / 25-0038-42 | 1 µL |
| Human HLA-E | PE / Biolegend / 342604 | 1 µL |
| 7-AAD | PerCp-Cy5.5 / Beckmen Coulter / A07704 | 5 µL |

### Example 14: Analysis of anticancer activity of Log-NK-CIITA KO cells

### 14-1) Examination of cancer cell killing ability using Calcein-AM

To use cancer cells K562 (chronic myeloid leukemia), DU145 (prostate cancer), HepG2 (liver cancer), HCC1954 (breast cancer), MDA-MB-231 (breast cancer), MDA-MB-468 (breast cancer) and SKOV3 (ovarian cancer) as target cells, each type of cells was suspended at a concentration of 1 × 10⁶ cells/mL in 1 mL of an assay medium, which is 10% FBS-containing RPMI 1640 medium, and then treated with 30 µL of Calcein-AM (Invitrogen), followed by incubation in a CO₂ incubator at 37°C for 1 hour. After incubation, the cells were washed twice with assay medium and then prepared at a concentration of 1 × 10⁵ cells/mL. NK or Log-NK-CIITA KO cells as effector cells were suspended in assay medium at a concentration of 3 X 10⁶ cells/mL for an effector: target cell ratio of 3:1 or a concentration of 1 × 10⁶ cells/mL for an effector: target cell ratio of 1:1. 100 µL of the prepared effector cells were added to each of 3 wells of a round-bottom 96-well plate, and then 100 µL of target cells at a concentration of 1 × 10⁵ cells/mL were added to each well. Subsequent experimental procedures and measurement value calculation method were performed in the same manner as in Example 4.

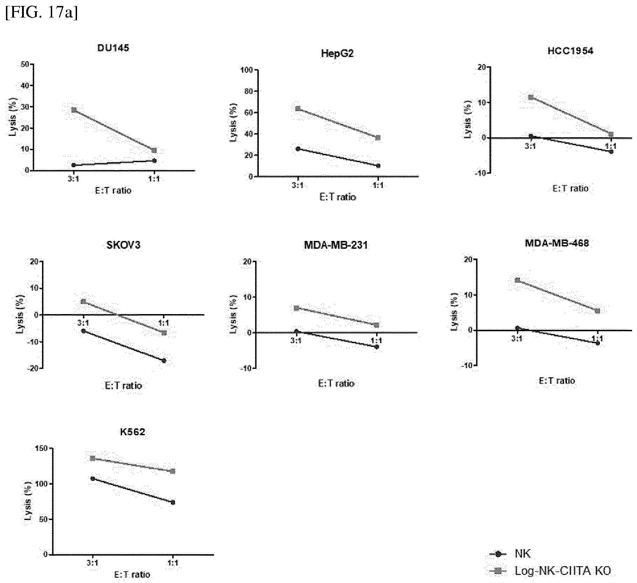

As shown in FIG. 17a, Log-NK-CIITA KO cells showed similar or increased cancer cell killing ability compared to the control NK cells against various cancer cell lines.

### 14-2) Examination of cancer cell killing ability using CFSE staining

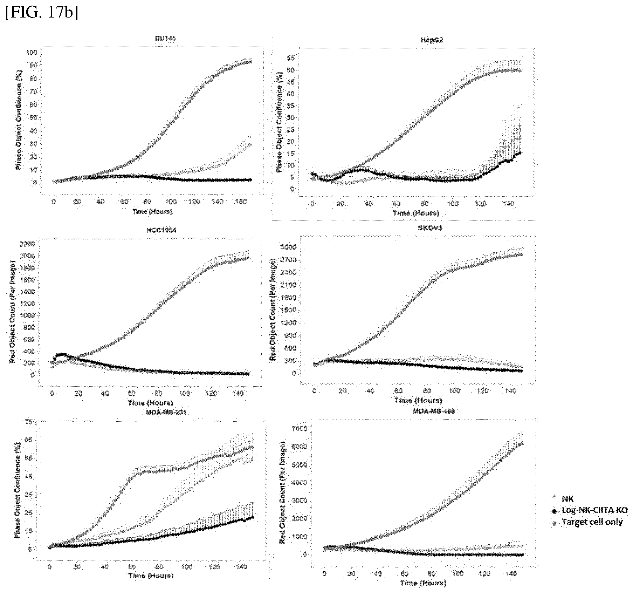

Each type of cancer cells (DU145, HepG2, HCC1954, MDA-MB-231, MDA-MB-468, and SKOV3) was suspended at a concentration of 4 × 10⁴ cells/mL in an assay medium, which is 10% FBS-containing RPMI 1640 medium, and then 100 µL of the cell suspension was added to each well of a 96-well plate and cultured for 18 to 22 hours, and then effector cells were added thereto. NK or Log-NK-CIITA KO cells as effector cells were suspended in RPMI 1640 medium containing 10% FBS and 1000 IU IL-2 at a concentration of 8 × 10⁴ cells/mL for an effector: target cell ratio of 2:1, and then 100 µL of target cells were added to each of 3 wells of the 96-well plate being cultured. While the cells were cultured in a CO₂ incubator at 37°C for 7 days, cell images were stored every 2 hours using a real-time cell image analyzer IncuCyte^{®}S3 system (Sartorius) provided inside the incubator, and the cancer cell killing ability of the cells was evaluated by analyzing the cell images. As a result, it was confirmed that Log-NK-CIITA KO cells showed increased cancer cell killing ability compared to the control NK cells against DU145 and MDA-MB-231, and showed killing ability similar to the control NK cells against other cancer cell lines (FIG. 17b). The above results show that even though the gene was edited and introduced to produce Log-NK-CIITA KO cells, the cancer cell killing ability of NK cells themselves did not decrease.

### Example 15: Knockout of B2M and CIITA genes using various gRNA delivery methods

In order to further confirm whether the same knockout effect is exerted even with a delivery system other than the shuttle, an RNP of B2M gRNA or CIITA gRNA and MAD7 nuclease was delivered to NK cells by various methods to confirm the efficiency of knockout of each gene. For the B2M gene, #8 gRNA (SEQ ID NO: 8) was used, and for the CIITA gene, #25 gRNA (SEQ ID NO: 44) was used. The same number of NK cells (1 × 10⁶ cells) was used for each delivery method. On the day of knock-out, cells were suspended in 500 µL of opti-MEM (Gibco, 31985062) medium and cultured in a 12-well plate, and the next day, 1 mL of NK cell culture medium was added to each well.

In Method 1, 26 µL of PBS, 4 µL of 40 µM MAD7 nuclease and 2 µL of 100 µM B2M or 2 µL of CIITA gRNA were mixed together and allowed to react at room temperature for 5 minutes or more, thus preparing an RNP reaction solution. 2 µL of 5 µM FSD64d1 shuttle was added to 48 µL of α-MEM medium and then mixed with 50 µL of the RNP reaction solution. Next, NK cells were treated with the mixture and incubated at room temperature for 1 minute and 30 seconds. The detailed delivery method was performed in the same manner as described in Example 1.

In Method 2, gRNA was delivered to NK cells by electroporation, and Nucleofector (Lonza) system and Human Natural Killer Cell Nucleofector Kit (Lonza, VPA-1005) were used. The same amounts of gRNA and MAD7 nuclease as in Method 1 and NK cells were added to 100 µL of the solution provided in the kit, and the mixture was placed in a cuvette and then subjected to electroporation using the U-01 program.

In Method 3, Lipofectamine CRISPRMAX transfection reagent (Invitrogen, CMAX00008) was used. 1 × 10⁶ NK cells were pre-suspended in a 12-well plate, and the same amounts of gRNA and MAD7 nuclease as in Method 1, 2.5 µL of Cas9 plus reagent, and 25 µL of opti-MEM were added to a 1.5-mL tube, thus preparing tube 1 which was allowed to react. 1.5 µL of CRISPRMAX reagent and 25 µL of opti-MEM were added to another 1.5-mL tube, thus preparing tube 2. Next, the reaction solution prepared in tube 1 was mixed with tube 2, the mixture was allowed to react at room temperature for 5 minutes, and then the NK cells were treated with the mixture.

In Method 4, Lipofectamine2000 transfection reagent (Invitrogen, 11668027) was used. 1 × 10⁶ NK cells were pre-suspended in a 12-well plate, and the same amounts of gRNA and MAD7 nuclease as in Method 1, and 25 µL of opti-MEM were added to a 1.5-mL tube, thus preparing tube 1. 2.5 µL of Lipofectamine2000 and 25 µL of opti-MEM were added to another 1.5-mL tube, thus preparing tube 2. Next, the reaction solution prepared in tube 1 was mixed with tube 2, the mixture was allowed to react at room temperature for 5 minutes, and then the NK cells were treated with the mixture.

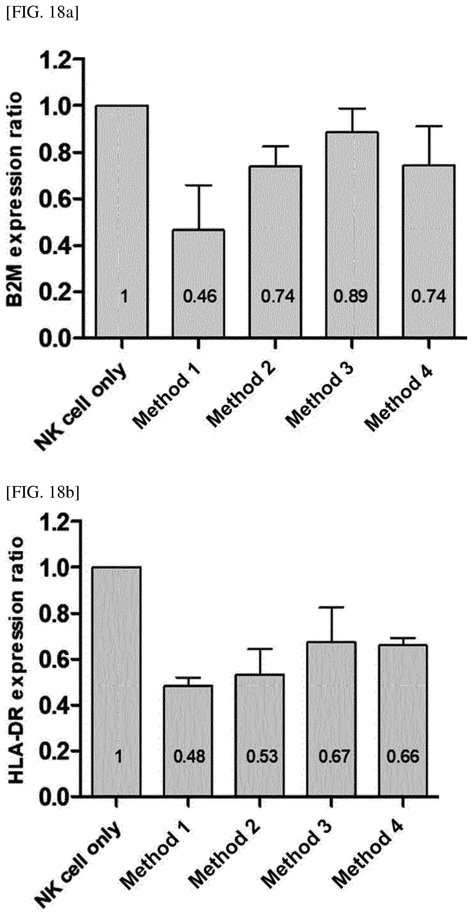

To confirm decreases in gene expression, the NK cells knocked-out of the B2M gene were analyzed by flow cytometry using B2M antibody after 3 days of culture, and the NK cells knocked-out of the CIITA gene were analyzed by flow cytometry using HLA-DR antibody after 7 days of culture. The gene expression level in wild-type NK measured by flow cytometry was converted to 1, and then the expression level in the NK cells knocked-out of the B2M or CIITA gene was calculated as a ratio relative to the expression level in the wild-type NK cells, thereby determining the efficiency of gene expression reduction. As shown in FIG. 18a, when the B2M expression level in the control NK cells was assumed to be 1, the expression of B2M decreased to 0.46 in Method 1, 0.74 in Method 2, 0.89 in Method 3, and 0.74 in Method 4, and there was no statistically significant difference in expression level decrease value between the delivery methods. As shown in FIG. 18b, when the HLA-DR expression level in the control NK cells was assumed to be 1, the expression of HLA-DR was decreased to 0.48 in Method 1, 0.53 in Method 2, 0.67 in Method 3, and 0.66 in Method 4 by the knockout of CIITA, and there was no statistically significant difference in expression level reduction value between the delivery methods. From the results of Example 14, it was confirmed that the gene editing system including B2M gRNA or CIITA gRNA, used in the present invention, may be delivered to target cells using various delivery methods.

### Example 16: In vitro evaluation of low-immunogenicity of Log-NK-CIITA KO cells

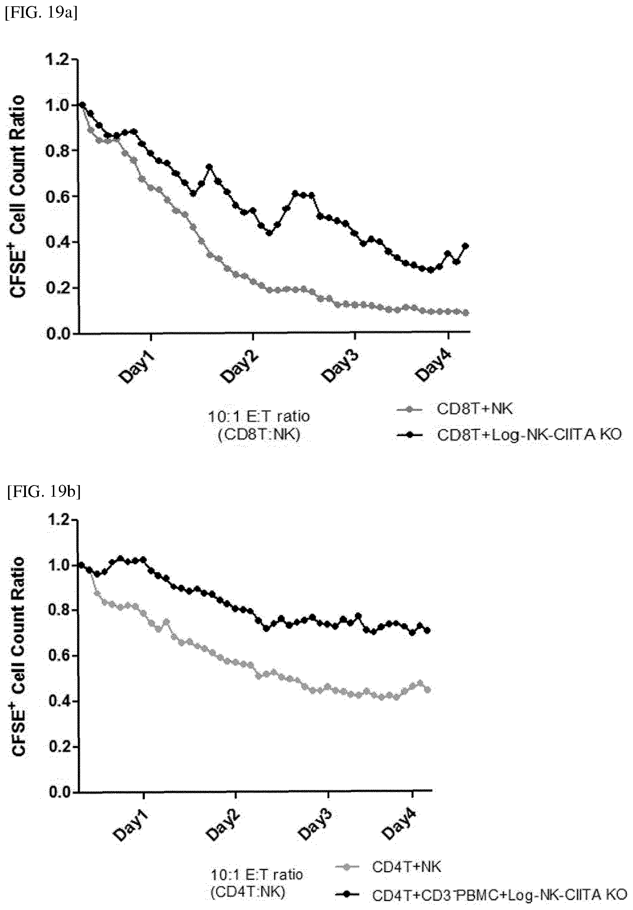

Log-NK-CIITA KO cells are NK cells having inhibited expression of type I HLA and type II HLA on the surface thereof, and thus were expected to have low immunogenic characteristics. In order to examine whether the cells can survive for a long time by evading the attack of the recipient's CD4 (+) T cells and CD8 (+) T cells when injected *in vivo,* an *in vitro* mixed lymphocyte reaction (MLR) assay was performed. In order to facilitate the MLR reaction, the experiment was conducted under conditions where the HLA-A, B, C and DRB1 types of CD8(+) T cells or CD4(+) T cells 100% mismatched with those of wild-type NK or Log-NK-CIITA KO cells and where the irradiated PBMCs used for culture of CD8(+) T cells or CD4(+) T cells were 37.5% identical with the HLA-A, B, C, DRB1 types of wild-type NK or Log-NK-CIITA KO cells. The method of isolating CD8(+) T cells from PBMCs was the same as the procedure described in Example 9, and in order to isolate CD4(+) T cells, a CD4(+) T cell isolation kit (Miltenyi Biotec, 130-096-533) was used and the method was as follows. PBMCs were washed once with MACS buffer, and then the cell pellet was suspended to a concentration of 1 × 10⁷ cells/40 µL, and biotin-antibody cocktail at a concentration of 1 × 10⁷ cells/10 µL was added thereto, followed by incubation at 4°C for 5 minutes. Thereafter, MACS buffer was added again to the cells to a concentration of 1 × 10⁷ cells/30 µL, and CD4(+) T cell microbead cocktail at a concentration of 1 × 10⁷ cells/20 µL was added to the cells, followed by incubation at 4°C for 10 minutes. After completion of incubation, the cell suspension was passed through an LS column (Miltenyi Biotec), and only cells expressing CD4 were isolated. PBMCs with 37.5% HLA type match were irradiated with 2,000 cGy and then mixed with the isolated CD4(+) T cells or CD8(+) T cells at a ratio of 1:1 ratio, followed by culture for 7 days. PBMCs were prepared in the same manner and mixed at a ratio of 1:1 with the CD4(+) T cells and CD8(+) T cells cultured for 7 days. The cells were further cultured for 7 days (a total of 14 days of culture) and used in the experiment. Control NK cells and Log-NK-CIITA KO cells were fluorescently stained with CFSE, and then CD8(+) T cells or CD4(+) T cells were diluted in the same manner as in Example 9 in order to make an effector cells: target cell ratio of 10:1, and the cells were added to a 96-flat-well plate. To the well to which CD4(+) T cells were added, the same number of PBMCs (which have been irradiated with 2,000 cGy and from which CD3 T cells have been removed) as CD4(+) T cells were added. While the 96-well plate prepared by adding cells thereto was incubated in a CO₂ incubator at 37°C, only cells positive for CFSE fluorescence were counted once every 2 hours using IncuCyte^{®}S3 system (Sartorius). As a result, as shown in FIG. 19a, it was confirmed that, when co-cultured with CD8(+) T cells for 4 days, the number of Log-NK-CIITA KO cells present was up to 5 times larger than the control NK cells. In addition, it was confirmed that, after Log-NK-CIITA KO cells were co-cultured with CD4(+) T cells and the irradiated PBMCs for 4 days, the number of Log-NK-CIITA KO cells present was 1.6 times larger than the control NK cells (FIG. 19b). These results suggest that Log-NK-CIITA KO cells can evade the attack of CD4(+) T cells and CD8(+) T cells with significantly higher efficiency than wild-type NK cells.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A composition for inhibiting immunogenicity of mammalian cells comprising, as an active ingredient, a nucleic acid molecule that inhibits expression of type II HLA protein.

2. The composition according to claim 1, wherein the nucleic acid molecule is a guide RNA (gRNA) which specifically recognizes a nucleotide sequence encoding the type II HLA protein or an activating protein thereof, or a nucleotide encoding the gRNA.

3. The composition according to claim 2, wherein the activating protein of the type II HLA protein is a class II major histocompatibility complex transactivator (CIITA) protein.

4. The composition according to claim 2, further comprising RNA-guided endonuclease or a nucleotide sequence encoding the RNA-guided endonuclease.

5. The composition according to claim 3, wherein the gRNA specifically recognizes a nucleotide sequence selected from the group consisting of SEQ ID NO: 20, SEQ ID NO: 22, SEQ ID NO: 24, SEQ ID NO: 27, SEQ ID NO: 29, SEQ ID NO: 30, SEQ ID NO: 40 and SEQ ID NO: 44, or a sequence complementary thereto.

6. The composition according to claim 1, further comprising a nucleic acid molecule that inhibits expression of β2-microglobulin protein.

7. The composition according to claim 6, wherein the nucleic acid molecule is a guide RNA (gRNA) which specifically recognizes a nucleotide sequence encoding the β2-microglobulin protein, or a nucleotide sequence encoding the gRNA.

8. The composition according to claim 7, further comprising RNA-guided endonuclease or a nucleotide sequence encoding the RNA-guided endonuclease.

9. The composition according to claim 7, wherein the guide RNA (gRNA) specifically recognizes a nucleotide sequence selected from the group consisting of SEQ ID NO: 1, SEQ ID NO: 8, SEQ ID NO: 9 and SEQ ID NO: 14, or a sequence complementary thereto.

10. The composition according to claim 4 or 8, wherein the RNA-guided endonuclease is selected from the group consisting of Cas9 (CRISPR associated protein 9), Cpf1 (CRISPR from Prevotella and Francisella 1) and MAD7.

11. The composition according to claim 1, further comprising a nucleic acid molecule encoding a type I HLA protein.

12. The composition according to claim 11, wherein the type I HLA protein is HLA-E protein.

13. The composition according to claim 1, wherein the cells are allogenic or autologous cells for transplantation.

14. The composition according to claim 13, wherein the cells are stem cells or immune cells.

15. The composition according to claim 14, wherein the immune cells are natural killer cells.

16. A hypoimmunogenic mammalian cell produced using the composition according to any one of claims 1 to 9 and 11 to 15.

17. A method for producing hypoimmunogenic mammalian cells comprising:
(a) inhibiting expression of a protein, selected from the group consisting of β2-microglobulin protein, type II HLA protein, an activating protein of type II HLA protein, and combinations thereof, in cells isolated from a mammalian subject; and
(b) introducing HLA-E gene into the cells.

18. The method according to claim 17, wherein step (a) is performed by introducing, into the cells, a guide RNA (gRNA) which specifically recognizes a nucleotide sequence encoding the protein, or a nucleotide sequence encoding the gRNA; and RNA-guided endonuclease or a nucleotide sequence encoding the RNA-guided endonuclease.

19. The method according to claim 18, wherein the guide RNA (gRNA) which specifically recognizes the nucleotide sequence encoding the β2-microglobulin protein specifically recognizes a nucleotide sequence selected from the group consisting of SEQ ID NO: 1, SEQ ID NO: 8, SEQ ID NO: 9, and SEQ ID NO: 14, or a sequence complementary thereto.

20. The method according to claim 17, wherein the activating protein of the type II HLA protein is a class II major histocompatibility complex transactivator (CIITA) protein.

21. The method according to claim 20, wherein the guide RNA (gRNA) which specifically recognizes the nucleotide sequence encoding the CIITA protein specifically recognizes a nucleotide sequence selected from the group consisting of SEQ ID NO: 20, SEQ ID NO: 22, SEQ ID NO: 24, SEQ ID NO: 27, SEQ ID NO: 29, SEQ ID NO: 30, SEQ ID NO: 40 and SEQ ID NO: 44, or a complementary sequence thereto.

22. The method according to claim 17, wherein step (b) is performed 2 to 4 days after completion of step (a).

23. The method according to claim 17, further comprising isolating cells with inhibited expression of the protein, after step (a).

24. A method of inhibiting immunogenicity of mammalian cells comprising a step of introducing the composition according to any one of claims 1 to 15 into the mammalian cells.
